(19) Europäisches Patentamt
European Patent Office
Office européen des brevets

(11) **EP 4 426 402 B1**

(12) **EUROPEAN PATENT SPECIFICATION**

(45) Date of publication and mention
of the grant of the patent:
**31.12.2025 Bulletin 2026/01**

(21) Application number: **22821764.2**

(22) Date of filing: **04.11.2022**

(51) International Patent Classification (IPC):
**A61M 16/00** *(2006.01)*

(52) Cooperative Patent Classification (CPC):
**A61M 16/0006; G16H 20/30; G16H 40/63;**
A61M 11/00; A61M 16/049; A61M 16/202;
A61M 2016/0024; A61M 2016/0027;
A61M 2205/3553; A61M 2205/3592;
A61M 2205/505; A61M 2205/52; A61M 2209/02;
A61M 2209/088; G16H 20/10;                (Cont.)

(86) International application number:
**PCT/US2022/048987**

(87) International publication number:
**WO 2023/081375 (11.05.2023 Gazette 2023/19)**

(54) **DEVICE FOR UNBLOCKING AND REMOVING SECRETIONS FROM AIRWAYS**

VORRICHTUNG ZUR BESEITIGUNG UND ENTBLOCKUNG VON SEKRETEN AUS ATEMWEGEN

DISPOSITIF DE DÉGAGEMENT ET D'ÉLIMINATION DES SÉCRÉTIONS DES VOIES
RESPIRATOIRES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **04.11.2021  US 202163275703 P**

(43) Date of publication of application:
**11.09.2024  Bulletin 2024/37**

(73) Proprietors:
• **Children's Hospital Medical Center**
**Cincinnati, Ohio 45229 (US)**
• **University of Cincinnati**
**Cincinnati, OH 45206 (US)**
• **B.G. Negev Technologies and Applications Ltd.,
at Ben-Gurion University
84105 Beer-Sheva (IL)**
• **Mor Research Applications Ltd.
6971054 Tel Aviv (IL)**

(72) Inventors:
• **GUTMARK-LITTLE, Iris
Cincinnati, OH 45237 (US)**
• **GUTMARK, Ephraim
Cincinnati, OH 45236 (US)**
• **CAVARI, Yuval
252 Nir Moshe (IL)**
• **KATOSHEVSKI, David
Omer (IL)**

(74) Representative: **Forresters IP LLP
Skygarden
Erika-Mann-Straße 11
80636 München (DE)**

(56) References cited:
WO-A1-2016/210266     WO-A1-2017/187390
WO-A1-2019/140333     US-A1- 2019 262 559

(52) Cooperative Patent Classification (CPC): (Cont.)
G16H 40/67

## Description

### Background and Summary of the Disclosure

The present disclosure relates to a device for treating obstructed airways. Specifically, the present disclosure relates to a device for treating the symptoms of diseases that cause persistent airflow limitation by applying air pressure oscillations and acoustic vibrations to the airways of a patient during treatment. The device may be used in a system that allows in-home treatment under remote supervision of a physician.

Patients with chronic obstructive pulmonary disease (COPD) suffer with persistent airflow limitation resulting from a combination of small airways, destruction of alveolar septa, and impaired secretions clearance. For example, chronic inflammation causes structural changes and the narrowing of small airways. Chronic inflammation also results in destruction of the lung parenchyma, which in turn leads to loss of alveolar attachments to the walls of the small airways and reduces their outward pulling and tethering, which normally keeps them open. These changes diminish the ability of the airways to remain open, particularly during expiration, especially in the dependent regions of the lung, which may become shut throughout the respiratory cycle and only opening with a deep inhalation, such as a sigh. Hypersecretion of mucus due to an increased number of goblet cells and enlarged submucosal glands may contribute to the tendency of the small airways to close up during part or all of the respiratory cycle, resulting in reduced alveolar ventilation of these lung regions. The disease further results in remodeling of the small-airway compartment and loss of elastic recoil by emphysematous destruction of parenchyma, resulting in a progressive decline of forced expiratory volume, inadequate lung emptying on expiration, and subsequent static and dynamic hyperinflation.

In addition to COPD, there are additional chronic supportive lung diseases that have different etiologies but share similar pathophysiology. The most prevalent example is cystic fibrosis (CF), a lethal genetic disease. Other diseases with similar pulmonary manifestations include non-cystic fibrosis bronchiectasis, primary and secondary immune deficiencies, primary ciliary dyskinesia, and more.

Currently, available therapies for patients include bronchodilators (e.g. LABA, LAMA), anti-inflammatory medications, oxygen, and non-invasive ventilation. Additionally, airway vibration techniques are available that improve mucus clearance and respiratory physical therapy to improve strength. However, the destruction of the normal anatomy of the small-airways and the loss of elastic recoil due to the destruction of parenchyma leads to reduced airway and alveolar potency and stability. The result of the above-mentioned process is reduced alveolar recruitment during inspiration and acceleration of premature small-airway collapse during a normal cough. As the disease progresses, the premature collapse will appear during normal expiration as well. These two symptoms amplify each other.

For example, premature airway collapse during cough leads to an ineffective clearance of secretion. As a result, less air reaches the lower airways and alveoli, causing low gas exchange in the infected areas and blocking the path for inhaled medication. The inhomogeneous pattern of lung damages results in an unequal air distribution throughout the lung, meaning that sicker areas are less ventilated than the healthier areas. Paradoxically, treating patients with aerosolized medications end up in an undesirable result: the healthier parts will receive more medication while the sicker parts hardly, if at all, receive medication.

This inequality poses the second challenge of delivering medication. The interaction between inflammation, tissue destruction, and poor secretion clearance places the patient in a vicious cycle. The poor drug delivery leads to excessive inflammation and mucus production that in turn leads to poor ventilation and poor mucus clearance, which further reduces the amount of drug delivered. Therefore, the overall ability to improve the pulmonary functions, the functional capacity, and perceived well-being of patients is suboptimal. Moreover, secondary complications such as COPD Exacerbation lead to rapid deterioration of health status, frequent hospitalizations, and premature death. Improvements in the treatment of such conditions are needed.

WO 2019/140333 A1 discloses a high frequency airway oscillation device, for internal airway vibration.

According to an illustrative embodiment of the present disclosure, a device for the removal of mucus from airways is disclosed. The device includes a housing defining an air passage, an air supply communicatively coupled to the air passage of the housing, and an acoustic generator coupled to the housing. The air supply is configured to provide air to the air passage of the housing. The acoustic generator is configured to provide acoustic oscillations to the housing. A combination of the air oscillations and the acoustic oscillations is configured to penetrate a mucus plug positioned in an air passage of a patient within 10 seconds.

According to another illustrative embodiment of the present disclosure, a device for the removal of mucus from airways is disclosed. The device includes a housing defining an air passage, an air assembly communicatively coupled to the air passage of the housing, and an acoustic generator coupled to the housing. The air supply assembly includes an air supply and a flow pulsating element, or an oscillating air supply configured to provide air oscillations to the air passage of the housing. The acoustic generator is configured to provide acoustic oscillations to the housing. The device has a first configuration for penetrating a mucus plug positioned in an air passage of a patient, wherein the first configuration includes a first air oscillation rate and a first acoustic oscillation rate. The device has a second configuration for removing mucus from the air passage of the patient, wherein the second configuration includes a second air oscillation rate and a second acoustic oscillation rate.

According to yet another illustrative embodiment of the present disclosure, a device for the removal of mucus from airways is disclosed. The device includes a housing with a nozzle defining an air passage, an air supply assembly communicatively coupled to the air passage of the housing, and an acoustic generator coupled to the housing. The air supply assembly includes an air supply and a flow pulsating element, or an oscillating air supply configured to provide air oscillations to the air passage of the housing. The acoustic generator is configured to provide acoustic oscillations to the housing. A combination of the air oscillations and the acoustic oscillations form a synthetic jet of air upon exiting the nozzle of the housing.

According to another illustrative embodiment of the present disclosure, a device for the removal of mucus from airways is disclosed. The device includes a housing defining an air passage, an air supply assembly communicatively coupled to the air passage of the housing, and an acoustic generator coupled to the housing. The air supply assembly includes an air supply and a flow pulsating element, or an oscillating air supply configured to provide air oscillations to the air passage of the housing. The acoustic generator is configured to provide acoustic oscillations to the housing. The device has a first configuration for use in a patient with active lung operation. The first configuration comprises a first air oscillation rate and a first acoustic oscillation rate. The device has a second configuration for use in a patient without active lung operation. The second configuration comprises a second air oscillation rate and a second acoustic oscillation rate, the second air oscillation rate being higher than the first air oscillation rate.

In a further illustrative embodiment of the present disclosure, a device for treating airways is disclosed. The device comprises a base unit, wherein the base unit includes a housing receiving an air pump, a circuit board assembly, and a memory. The device also comprises a hand unit operatively coupled to the base unit and including a housing supporting an acoustic transducer, and an acoustic duct in communication with the acoustic transducer. The acoustic transducer of the hand unit is configured to apply acoustic oscillations according to a predetermined pattern.

The device may further comprise a nebulizer coupled to the mouth piece of the device. The device may be Bluetooth capable. The device may be configured to connect to wireless Internet. The base unit may be configured to be mobile and worn by a patient. Where the base unit is wearable, the device may comprise a chest rack configured to hold the hand unit in a stationary position to facilitate hands-free use of the device.

A system may utilize the device, wherein the system comprises a first personal electronic application in communication with the device and in communication with a cloud-based server; and a second personal electronic application in communication with the cloud-based server. The second personal electronic application of the system may be able to control a variety of parameters of the device from a remote location via the cloud-based server and the first personal electronic application. The first personal electronic application may include a gamification feature.

In another illustrative embodiment of the present disclosure, a device for treating airways includes a base unit having a housing, wherein the housing receives an air pump, a circuit board assembly, and a memory. The device further includes a hand unit operatively coupled to the base unit and including a housing supporting an acoustic transducer, a central duct in fluid communication with the base unit, and an acoustic duct in fluid communication with the central duct. The acoustic transducer is configured to apply acoustic oscillations according to a predetermined pattern. The hand unit further includes a mouthpiece coupled to the housing.

The housing of the hand unit and/or the housing of the base unit may further contain a flow pulsating element configured to alter (e.g., reduce and/or interrupt) the continuous airflow stream from the air pump according to a predetermined pattern (e.g., abrupt (square pattern) or gradual (sinusoidal pattern)). The device may further comprise a nebulizer coupled to the mouthpiece of the device. The device may be configured for wireless communication (e.g., Bluetooth capable). For example, the device may be configured to connect wirelessly to the Internet. The base unit may be configured to be mobile and worn by a patient. Where the base unit is wearable, the device may comprise a chest rack configured to hold the hand unit in a stationary position to facilitate hands-free use of the device.

A system may utilize the device, wherein the system comprises a first personal electronic application in communication with the device and in communication with a cloud-based server; and a second personal electronic application in communication with the cloud-based server. The second personal electronic application of the system may be able to control a variety of parameters of the device from a remote location via the cloud-based server and the first personal electronic application. The first personal electronic application may include a gamification feature.

In yet another illustrative embodiment of the present disclosure, a method of treating diseased airways is disclosed, said method not being part of the invention. The method comprises the step of providing a device comprising a base unit, a hand unit, and a flow pulsating element disposed in either a housing of the hand unit or a housing of the base unit. The base unit comprises a housing containing an air pump, a circuit board assembly, and a memory. The hand unit is operatively coupled to the base unit and includes a housing containing an acoustic transducer, a central duct in fluid communication with the base unit, and an acoustic duct in fluid communication with the central duct. The operation of the device is controlled by a protocol stored on the circuit board assembly. The method further includes the steps of inserting the mouthpiece into the mouth of the patient; sensing breathing cycles for inhalation and exhalation stages of a respiratory cycle of the patient during tidal breathing; operating the air pump to provide air flow to the patient operating the flow pulsating element to alter (e.g., reduce and/or interrupt) the continuous airflow stream from the air pump according to a predetermined pattern (e.g., abrupt (square pattern) or gradual (sinusoidal pattern)); and operating the acoustic transducer to generate acoustic soundwaves.

The mouthpiece of the device may also include a nebulizer. Where the device includes a nebulizer, the method may further comprise the step of delivering medication to the patient via the nebulizer and the air flow duct. The method may further comprise the step of using the device to store data related to at least one of patient adherence, patient progress, protocol setting, and patient condition. Wherein data is stored, the method may further include the step of transmitting the stored data from the device to a cloud-based server. Wherein the data is transmitted, the method may further comprise the step of downloading stored data from the cloud-based server to a remote application device. Wherein the data is downloaded, the method may further comprise the step of adjusting the settings of the device using the remote application device.

Additional features and advantages of the present technology will become apparent to those skilled in the art upon consideration of the following detailed description of the illustrative embodiment exemplifying the best mode of carrying out the technology as presently perceived.

The present invention is defined by the attached claims.

## Brief Description of the Drawings

The detailed description of the drawings particularly refers to the accompanying figures in which:

FIG. 1A is a front view of the airway clearance device of the present disclosure, the device including a hand unit, a base unit, and a tube coupling the base unit and the hand unit;

FIG. 1B is a side view of the device of FIG. 1A in use by a patient, wherein the base unit is wearable on the body of a patient;

FIG. 1C is a side view of the device similar to FIG. 1B, showing a chest rack for mounting the hand unit to the body of the patient;

FIG. 2A is a diagrammatic view of an illustrative embodiment of a hand unit for use with the device of FIG. 1A;

FIG. 2B is a diagrammatic view of another illustrative embodiment of a hand unit for use with the device of FIG. 1A;

FIG. 3A is a diagrammatic view of another illustrative embodiment of a hand unit for use with the device of FIG. 1A;

FIG. 3B is a diagrammatic view similar to FIG. 3A, wherein the hand unit does not include a speaker positioned with the hand unit of the device;

FIG. 4 is a diagrammatic view of an illustrative system in which the device of FIG. 1A may be utilized, allowing a patient and his or her physician to monitor use of the device and data generated through the use of the device;

FIG. 5 is an illustration of an application for a handheld device that may be used within the illustrative system of FIG. 4 to complement the use of the device of FIG. 1A, including five illustrative features of the application;

FIG. 6A is a graphical representation of the amplitude of air pressure, the amplitude of sound pressure, the frequency of air pressure, and the frequency of sound pressure over the time duration of three modes of operation of the device of FIG. 1A;

FIG. 6B is a table displaying the protocol characterization of various settings of the device of FIG. 1A;

FIG. 7A is a diagrammatic view and exemplary data related to a general branched airway of a patient;

FIG. 7B is a schematic illustration of a closed-loop system of the device of FIG. 1A for determining the resistance of varied branched airways;

FIG. 8 is a schematic view of a test setup for testing the effects of oscillating air pressure pulses and acoustic oscillations on a simulated airway;

FIGS. 9A-9D are illustrations of simulated mucus removal from the simulated airway during testing of the test setup of FIG. 8;

FIGS. 10A-10C are a plurality of graphs comparing the effects of oscillating air pressure pulses and acoustic oscillations on simulated mucus during testing of the test setup of FIG. 8;

FIGS. 11A-11D are a plurality of graphs illustrating the presence of and movement of simulated mucus within the simulated airway of FIG. 8 during testing;

FIGS. 12A-12D provide a plurality of graphs illustrating the time to penetration of simulated mucus based on the speaker frequency (Hz), the solenoid frequency (BPM), and air pressure (cmH$_2$O) within the simulated airway of FIG. 8 during testing;

FIG. 13 is a flow chart to illustrate to illustrate the cause and effect of device geometry, air oscillation, and acoustic oscillation on the simulated airway of FIG. 8;

FIG. 14 is a schematic view of a test setup for testing the effects of oscillating air pressure pulses and acoustic oscillations on a simulated airway, wherein the test setup includes a 3/2 solenoid valve and a microphone positioned 2.54 cm away from the speaker;

FIGS. 15A and 15B are graphs illustrating the sound effects of a 3/2 solenoid valve at varied predetermined speaker amplitude values and a consistent valve frequency and the effects of a 3/2 solenoid valve at varied predetermined valve frequencies and a consistent speaker amplitude at a consistent air pressure of 30 cmH2O;

FIGS. 15C and 15D are tables illustrating the sound effects of a 3/2 solenoid valve at varied predetermined speaker amplitude values and varied predetermined solenoid valve frequencies at a consistent air pressure of either 20 cmH2O or 40 cmH2O;

FIG. 16 is a test setup similar to the test setup of FIG. 14, wherein the microphone is positioned a distance of 1 meter away from the speaker;

FIGS. 17A and 17B are graphs illustrating the speaker sound pressure (kPa) over time and the speaker sound level (Db) over frequency of the test setup of FIG. 16;

FIG. 18A is a test setup similar to that of FIG. 14, wherein the microphone is positioned at a nozzle exit and without foam over the speaker;

FIG. 18B is a test setup similar to that of FIG. 14, wherein the microphone is positioned at a speaker exit and the speaker is encased in foam;

FIGS. 19A and 19B are graphs illustrating the speaker sound pressure (kPa) over time and the speaker sound level (dB) over amplitude for the test setup of FIG. 18A;

FIG. 19C and 19D are graphs illustrating the speaker sound pressure (kPa) over time and the speaker sound level (dB) over amplitude for a test setup similar to that of FIG. 18B, wherein the speaker is encased in foam;

FIGS. 19E and 19F are graphs illustrating the speaker sound pressure (kPa) over time and the speaker sound level (dB) over amplitude for a test setup similar to that of FIG. 18A without foam over the speaker;

FIG. 19G and 19H are graphs illustrating the speaker sound pressure (kPa) over time and the speaker sound level (dB) over amplitude for the test setup of FIG. 18B, wherein the speaker is encased in foam;

FIGS. 20A-23B are graphs illustrating the movement of a mucus simulant within the test setup of FIG. 14 utilizing varying frequencies;

FIGS. 24A-25B are images illustrating the movement of a mucus simulant within the test setup of FIG. 14 at a speaker amplitude of 0.4 and a 3/2 valve frequency of 12 Hz;

FIGS. 26A-27B are images illustrating the movement of a mucus simulant within the test setup of FIG. 14 at a speaker amplitude of 0.4 and a 3/2 valve frequency of 15 Hz;

FIGS. 28A-29B are images illustrating the movement of a mucus simulant within the test setup of FIG. 14 at a speaker amplitude of 0.4 and a 3/2 valve frequency of 20 Hz;

FIGS. 30A-31B are images illustrating the movement of a mucus simulant within the test setup of FIG. 14 at a speaker amplitude of 0.4 and a 3/2 valve frequency of 30 Hz;

FIG. 32 is a schematic view, similar to FIG. 14, of an illustrative test setup for testing the effects of oscillating air pressure pulses and acoustic oscillations on a simulated airway, wherein the test setup includes a Percussionaire® IPV device;

FIG. 33 is a schematic view, similar to FIG. 32, of an illustrative test setup for testing the effects of acoustic oscillations on a simulated airway, wherein the test setup includes a Vibralung acoutstical percussor;

FIG. 34 is a graph illustrating recorded pressures (20cmH2O) at the first, upstream pressure sensor and the second, downstream pressure sensor over time for the illustrative test setup of FIG. 32;

FIGS. 35A and 35B are graphs illustrating recorded pressures (20cmH2O) at the first, upstream pressure sensor and the second, downstream pressure sensor over time for the illustrative test setup of FIG. 33;

FIG. 36 is a graph illustrating recorded pressures (20cmH2O) at the first, upstream pressure sensor and the second, downstream pressure sensor over time for the illustrative test setup of FIG. 14;

FIG. 37 is a graph illustrating frequency response of the illustrative test setup of FIG. 14;

FIG. 38 is a diagrammatic view of an illustrative airway clearance device of the present disclosure, the device including a hand unit, a base unit, and a tube coupling the base unit and the hand unit;

FIG. 39 is a front perspective view of the base unit of FIG. 38;

FIG. 40 is a right side perspective view of the base unit of FIG. 39, with the cover removed to show internal components;

FIG. 41 is a left side perspective view of the base unit of FIG. 39, with the cover removed to show internal components;

FIG. 42 is a diagrammatic view of a hand unit of FIG. 38;

FIG. 43 is a is a schematic view of a test setup for testing the effects of oscillating air pressure pulses and acoustic oscillations on a simulated airway, the test setup including the airway clearance device of FIG. 38;

FIG. 44 is a graph showing pressure traces at the hand unit of FIG. 38 and near the simulated lung of the test setup of FIG. 43;

FIGS. 45A-45D are illustrations of simulated mucus removal from the simulated airway during testing of the test setup of FIG. 43 and for the same conditions as FIG. 44; and

FIG. 46 is a graph showing pressure traces from tests with conventional devices.

## Detailed Description of the Drawings

The embodiments of the disclosure described herein are not intended to be exhaustive or to limit the disclosure to the precise forms disclosed. Rather, the embodiments described herein enable one skilled in the art to practice the disclosure.

The illustrative device of the present disclosure is a non-invasive, handheld inhalation device utilized to provide lung expansion through positive expiratory pressure (PEP) and secretion clearance through creation of vibrations in the airways resulting from a combination of acoustic and air pressure oscillations, which may result in effective personalized drug delivery into the small-airways on a daily basis treatment. The device may further be used to promote bronchial drainage, airway clearance and expectoration.

The device may be used by a patient who can properly self-administer in a variety of settings after training and under the care of a physician for daily treatment outside of a medical facility. For example, the patient can self-administer in the home, a hospital during short or extended stays whether supervised or unsupervised, a nursing facility, a sub-acute facility, or in another remote location. The patient may also use the device with the assistance of a physician in a hospital, lab, office setting, or other medical setting for monitoring and optimizing the medical therapy. Specifically, the device is intended to be used as a portable device for single-patient use for multiple uses as a non-sterile device. In an exemplary treatment plan, a patient may use the device two or three times per day, with each treatment duration lasting from about 20 to about 25 minutes, depending on physician instructions. For example, a physician may otherwise instruct the patient to use the device only once per day or more than three times per day for a treatment duration lasting shorter than 20 minutes or longer than 25 minutes.

In an illustrative embodiment, the device includes preprogrammed protocols for three preset modes that represent patient conditions, specifically corresponding to a "mild" condition, a "moderate" condition, and a "severe" condition. Other preprogrammed protocols can be imagined. Alternately, the device may be programmable to include different settings. When settings are chosen, the device is software-controlled to define frequency, acoustic/pressure amplitude, and duration of oscillation with a special algorithm. The illustrative device further includes a positive expiratory pressure (PEP) mechanism for creating back-flow resistance, which may expand the lungs and expand and help hold open the airways. PEP is applied as the patient breathes through the mouthpiece. A conventional PEP may use automatic pressure level (APL) valves. The device may further include a nebulizer to efficiently deliver aerosol drugs.

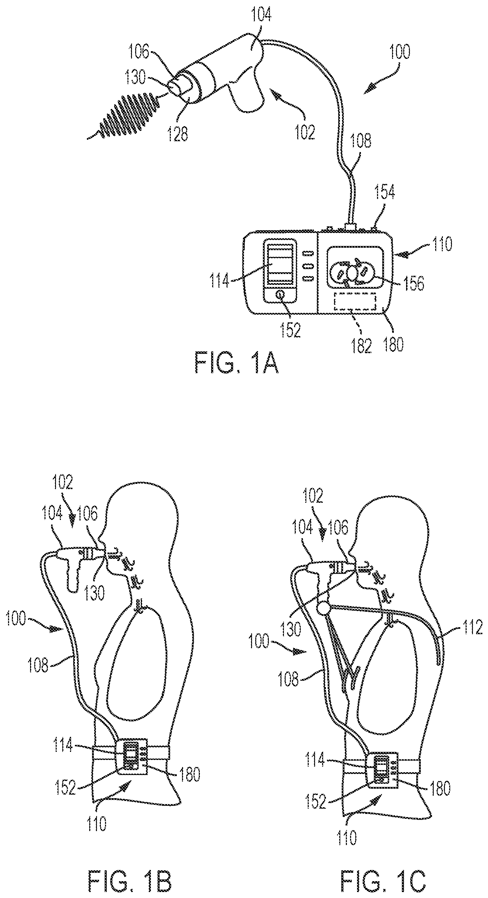

Referring specifically to FIGS. 1A-1C, an illustrative device 100 is shown. The device includes a hand unit 102 comprised of a housing 104 and a mouthpiece 106 coupled to the proximal end of the hand unit 102. The housing 104 of the hand unit 102 is illustratively comprised of a polymer, such as hard plastic, but may be comprised of other rigid materials in other embodiments. The device 100 further comprises a base unit 110. The base unit 110 and the hand unit 102 are illustratively placed in fluid communication via a flexible air tube 108. In an illustrative embodiment, the air tube 108 is corrugated. In other embodiments, the air tube 108 may not be corrugated.

The illustrative base unit 110 is configured to provide positive and oscillated air pressure flow to the hand unit 102. The base unit 110 is comprised of a housing 180, an air flow generator or air pump 182, a custom printed circuit board assembly (not shown) with control and pressure measurement components operatively coupled to a microprocessor (not shown), an indicator light (i.e., a light-emitting diode) 154, a power switch 152, a memory (not shown) to record usage and therapy parameters, a first pressure sensor (not shown), a second pressure sensor (not shown), and a flow pulsating element 156 that creates oscillating air pressure pulses. The flow pulsating element 156 is illustratively configured to alter (e.g., reduce and/or interrupt) the continuous airflow stream from the air pump 182 according to a predetermined pattern (e.g., abrupt (square pattern) or gradual (sinusoidal pattern)). The base unit 110 may also include an alternate current input connector (not shown) to plug into a standard 120 volt AC and/or a rechargeable battery pack (not shown) to provide power to the base unit 110. The base unit 110 further includes a display screen 114 that allows the patient and/or physician to monitor and receive information regarding the device operation mode, usage time, and other treatment parameters. In an illustrative embodiment, the display screen 114 may be a touch screen, and, specifically, a liquid crystal display touch screen.

In an illustrative embodiment, the base unit 110 can be intended for use as a desktop unit as shown in FIG. 1A. In illustrative embodiments shown in FIGS. 1B and 1C, the base unit 110 may be used as a wearable device (e.g., secured to a patient's waist via a belt or strap). In an illustrative embodiment of FIG. 1C, the hand unit 102 may be mounted on a chest rack 112 so that the patient can use the device 100 hands free, thus preventing hand fatigue and enhancing patient compliance with use of the device.

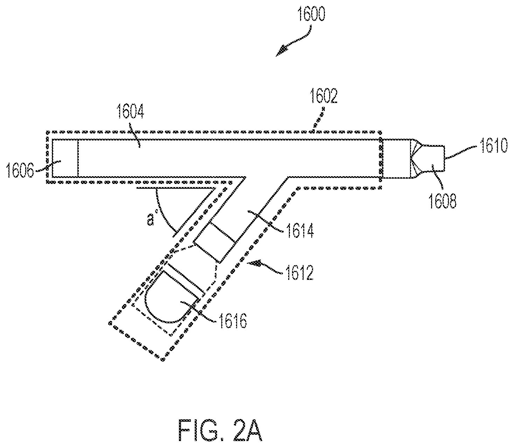

FIG. 2A provides a diagrammatic view of illustrative hand unit 1600 similar to hand unit 102 described above but where the acoustics may be introduced after the air pulsations. The exemplary hand unit 1600 includes a housing 1602 containing an interior tube or central duct 1604 with a standardized fluid fitting 1606 on one end and a mouthpiece 1608 on an opposite end. The standardized fluid fitting 1606 is configured to couple with another tube, such as a corrugated tube 108, which couples to the base unit 110 of the device 100 (for example, as shown in FIG. 1A).

Still referring to FIG. 2A, a branch 1612 of the hand unit 1600 extends from a side of the hand unit 1600 at an angle (illustrative an acute angle) relative to the interior tube or central duct 1604 of the hand unit 1600. The branch 1612 is contained within the housing 1602 and includes a branch tube or acoustic duct 1614 fluidly coupled to the interior tube 1604, and an acoustic driver, such as a speaker module or acoustic transducer 1616, so that acoustic waves generated by the speaker module 1616 may travel into the interior tube 1604. The interior tube or central duct 1604 is in fluid communication with an airflow duct 1610 of the mouthpiece 1608, and the acoustic duct 1614 is interconnected with the central duct 1604, which allows the flow of air and the flow of acoustic waves to efficiently be delivered directly into the mouth of the patient.

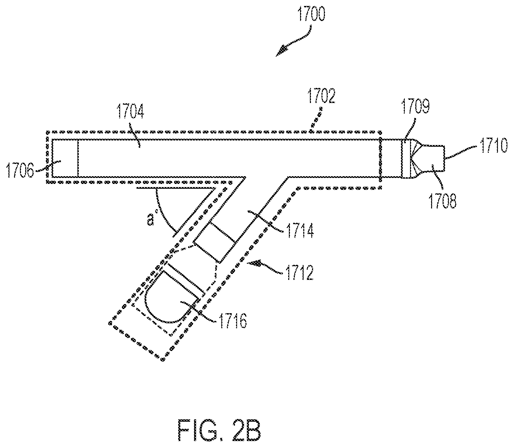

FIG. 2B provides a diagrammatic view of another illustrative embodiment of a hand unit 1700 for use with the device 100 as described above. In such an embodiment, a hand unit 1700 includes a housing 1702 with an interior tube or central duct 1704 and a standardized fluid fitting 1706 on one end and a mouthpiece 1708 on an opposite end. The standardized fluid fitting 1706 is configured to couple with another tube, such as a corrugated tube 108, which couples to the base unit 110 of the device 100 (FIG. 1A). Illustratively, an interior valve 1709 may be installed upstream of the hand unit 1700 in the base unit 110 (FIG. 1A) or is otherwise integrated into the hand unit 102. In certain illustrative embodiments, the interior (or exterior) valve 1709 may be included as part of the mouthpiece 1708. The interior valve 1709 may be a two-way valve that allows for passage of the pulsed air flow during inhalation along an inhalation pathway during an inhalation event, and/or the two-way valve may close the inhalation pathway and open an outlet to allow for passage of the exhaled air during an exhalation event. In other embodiments, a three-way valve may be used. The hand unit 1700 further includes a branch 1712 and any related components as described above, including a branch tube or acoustic duct 1714 and a speaker module or acoustic transducer 1716. The interior tube or central duct 1704 is in fluid communication with an airflow duct 1710 of the mouthpiece 1708, and the acoustic duct 1714 is interconnected with the central duct 1704, which allows the flow of air and the flow of acoustic waves to efficiently be delivered directly into the mouth of the patient.

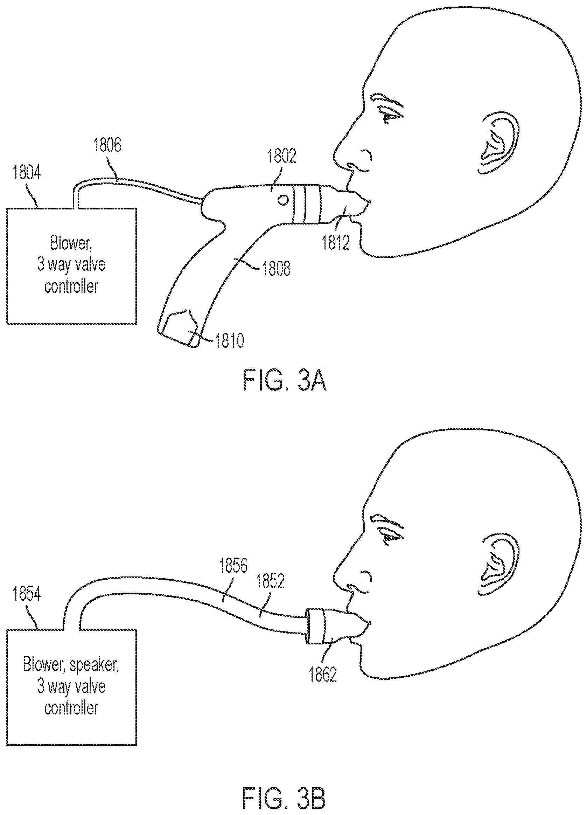

As shown by the diagrammatic illustrations in FIG. 3A and 3B, a minimalist hand unit may be also be utilized. For example, FIG. 3A illustrates a hand unit 1802 similar to those described above, wherein the base unit 1804 includes an air blower, a three-way valve, a controller, and a tube 1806 to operatively couple the base unit 1804 to the hand unit 1802. The hand unit 1802 includes a branch 1808 containing the speaker 1810 to generate acoustic waves and a mouthpiece 1812 for use by the patient.

Comparatively, FIG. 3B illustrates a hand unit 1852 fluidly coupled to a base unit 1854 including an air blower, a three-way valve, a controller, and a speaker. The hand unit 1852 illustratively includes only a tube 1856 having a mouthpiece 1862 to place a patient in fluid communication with the base unit 1854. The hand unit 1852 may not include a housing and does not include a branch portion or a speaker, so that the hand unit 1852 is consistent with the tube 1856.

In any of the above described embodiments, the speaker may be enclosed in a casing that includes materials to dampen the ambient sound while maintaining therapeutically effective sound levels at the outlet of the mouthpiece as discussed further herein.

Illustrative examples of air pulsation elements 156 of the device 100 include electrically operable valves (e.g., solenoid valves) or custom designed air pulsation units that generate pulsed air flow at specific frequencies upon application of constant air flow at the inlet of the air pulsation unit. The illustrative flow pulsating element 156 may be mounted either in the hand unit 102 or the base unit 110, and pulses the continuous airflow stream from the air pump 182, thus creating air pressure pulses or vibrations in a frequency range between about 5 Hz and about 700 Hz. The flow pulsating element 156 may be implemented as either a custom design or an off-the-shelf item, such as a solenoid valve. The custom printed circuit board assembly and microprocessor controls and displays parameters such as air pulse amplitude and frequency, air pump pressure and flow rate, flow pulsating element frequency and rotating velocity, first pressure sensor and second pressure sensor readings, and air pump temperature. The flow pulsating element 156 may also create a centrifuging vortex flow wherein the axis of rotation is in the direction of the airflow to increase the ability to efficiently and promptly penetrate an obstruction in an airway formed by mucus secretion.

Examples of illustrative flow pulsating elements 156 include, for example, an electrically operable valve (e.g., a solenoid valve). The flow pulsating elements 156 of the present invention, according to a first alternative, include a three-way solenoid valve, which allows a downstream flow of air during a first part of the device cycle and an upstream flow of air during a second part of the device cycle, which allows the device to send the pulsations into the patient during an inhalation event while also allowing for expiration of air during an exhalation event. According to a second alternative of the present invention, two high-speed one-directional valves are utilized as a flow pulsating element, wherein the valves consist of a first directional valve for inflow and a second directional valve for outflow to fulfill the needs described above.

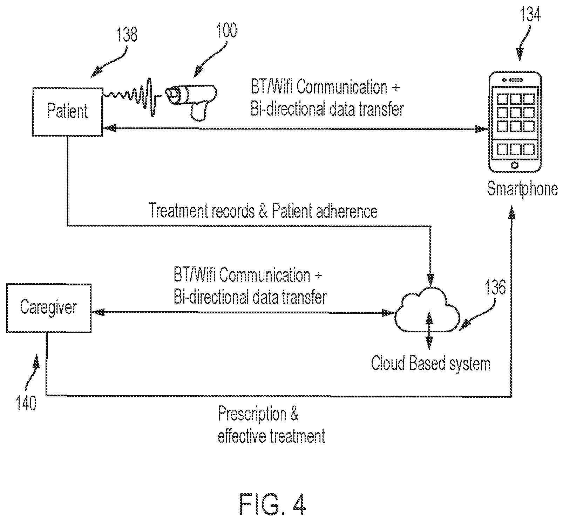

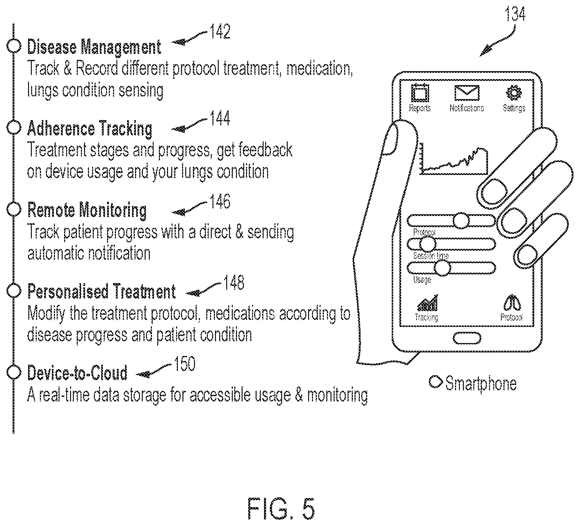

The device 100 may be integrated into an illustrative system as portrayed by FIGS. 4 and 5. For example, after daily use a patient 138 may wirelessly communicate with the device 100 via a smartphone 134 utilizing an application to download and transfer the stored treatment and other therapy and usage records from the device 100 to a cloud-based server 136. For example, the device 100 may transmit the patient's adherence tracking records, protocol setting, patient condition, and disease progress. The smartphone 134 may wirelessly communicate with the device 100 through an application such as Bluetooth or over a WiFi or data connection to allow bi-directional data transfer. The smartphone 134 may then be put into communication with the cloud-based system 136 through an application such as Bluetooth or over a WiFi or data connection to allow bi-directional data transfer.

With further reference to FIG. 4, a treating physician 140 may communicate remotely with the cloud-based server 136 using a graphic user interface application to review the patient's records and evaluate the available monitored data to optimize the medical therapy and usage records or to configure and apply patient-specific therapy parameters. For example, the graphic user interface application may be applied to a tablet, smartphone, personal computer, or other computer to communicate with the cloud-based system 136 through an application such as Bluetooth or over a WiFi or data connection to allow bi-directional data transfer. To configure and apply patient-specific therapy parameters, the physician 140 may upload such parameters to the cloud-based system 136, which the patient 138 may then download to the smartphone 134 to update the device 100 over the connection described above. To facilitate patient adherence, the application may include a gamification feature; for example, the application may be presented to the patient as a virtual reality gaming system.

Referring specifically to FIG. 5, the application on the smartphone 134 illustratively includes a special algorithm that enables five features: disease management 142, adherence tracking 144, remote monitoring 146, personalized treatment 148, and device-to-cloud 150. The disease management feature 142 tracks and records different protocol treatments, medication, and the condition of the patient's lungs. The adherence tracking feature 144 tracks and records treatment stages and progress and receives feedback on the device usage and the condition of the patient's lungs. The remote monitoring feature 146 tracks the patient's progress and sends automatic notifications. The personalized treatment feature 148 allows modification of the treatment protocol and medication according to the disease progress and the condition of the patient. The device-to-cloud feature 150 allows real-time data storage for accessible usage and monitoring by the physician or a caregiver.

To facilitate usage of the above-described application, the illustrative device 100 collects and stores to memory the patient's data, producing a database containing indication of the condition of the lungs, usage records, protocol setting, patient compliance, and treatment progress. For example, during a patient's tidal breathing in treatment, the device 100 senses the base-line breathing cycles by determining the patient's stages of inhalation and exhalation, e.g., amplitude, frequency, timing, and the ratio between inspiratory and expiratory breathing. The accumulated records may then be used to analyze the patient's lung conditions and trends during treatment over a period of time and usage of the device 100. The sensing analysis/monitoring ideally takes place within a short amount of time, e.g. between 1 and 5 seconds, before the treatment mode begins to automatically adjust treatment protocol selection according to the accumulated patient records and analysis. The device 100 may then wirelessly transmit notifications to the caregiver or physician indicating patient adherence, treatment progress, and lung condition. The device 100 may also provide a recommendation on personalized treatment, such as treatment duration and frequency, pressure and/or sound amplitude, positive expiratory pressure, and timing of aerosolized medication.

The first pressure sensor and the second pressure sensor may cooperate to observe, register, and transmit feedback from the patient relating to airway resistance and airway clearance throughout treatment. One of the first pressure sensor or the second pressure sensor is positioned to register air pressure during inhalation and the other of the first pressure sensor and the second pressure sensor is positioned to register air pressure during exhalation. The registered air pressure of the first pressure sensor and the second pressure sensor is compared, and the registered air pressure of each pressure sensor between a breath and the subsequent breaths is compared to determine the change in airway resistance during clearance of the airway. The information may be utilized to adjust the device parameters, either manually or automatically in an open loop fashion or a closed loop algorithm, to optimize airway clearance.

The illustrative device 100 applies a combination of oscillated air pressure and acoustic sound pulses superimposed over the normal respiratory waveforms to travel throughout the lungs via a conducting airway system. The vibrations propagate into the chest cavity using air as a carrier medium to travel throughout the lungs.

As briefly discussed above, the illustrative device 100 applies oscillating positive expiratory pressure (PEP) via combination of breathing against a positive pressure source as well as breathing through a regulated exhalation port. This reduces the collapse of small-airways during expiration and allowing improved escape of air during expiration by bypassing the collapsed small-airways and thereby reducing hyperinflation. Breathing against PEP may result in increased expiratory time, which in turn leads to a smaller exhaled volume, increasing the lung volumes. Breathing against PEP for a prolonged period of time may also improve gas exchange. PEP treatment may also decrease the pressure drop across the airway wall, reducing airway collapse, and encourages coughing and improvement of airway clearance. By reopening the airway and improving the respiratory pattern through PEP, drug delivery into the small-airway may be enhanced due to deeper drug penetration and enhancement of peripheral aerosol distribution.

Typical daily treatment using the illustrative device 100 may comprise 2 to 4 treatments for a duration of about 15 to 30 minutes per each treatment, with a resting time of about 3 hours between each treatment. Differences in patients and physician preferences may result in variations from the defined typical daily treatment. For example, treatment may comprise 1 treatment per day, or treatment may comprise more than 4 treatments per day.

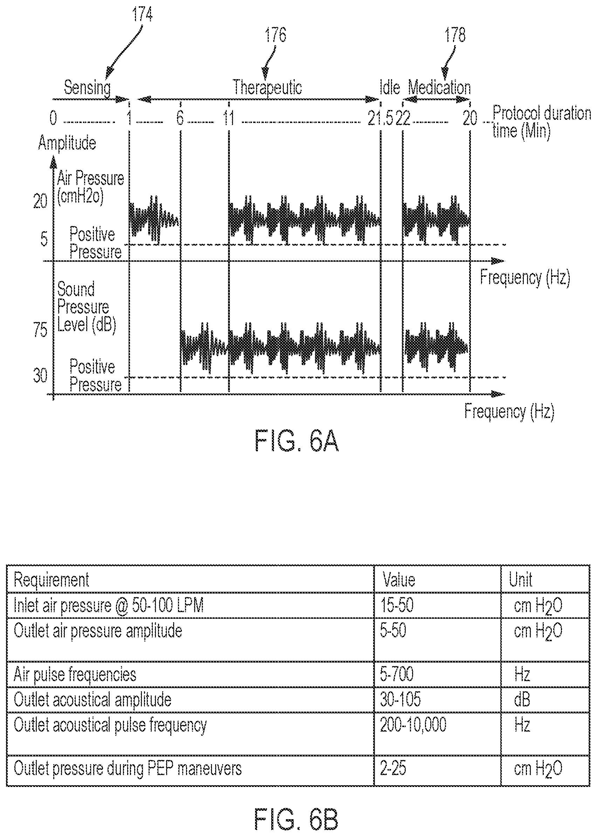

Treatment duration may last for fewer than or greater than 15 to 30 minutes per treatment, and the resting time between treatments may be less than or greater than a time period of 3 hours. Illustratively, the device 100 includes preprogrammed protocols for three preset modes representing patient conditions: mild, moderate, and severe. Each protocol includes modes of operation as demonstrated by FIGS. 6A and 6B.

For example, the illustrative device 100 includes a sensing mode of operation 174. During the sensing mode of operation, the device 100 senses the base line breathing cycles for inhalation and exhalation stages of the patient's respiratory cycles during tidal breathing, including expiratory and inspiratory timing, rate, and ratio. The illustrative device 100 may also conduct a lung compliance evaluation during this mode of operation. A special machine learning algorithm may analyze the patient condition, and then monitors trends regarding treatment progression based on present and past performance, which allows the algorithm to accordingly adjust the device 100 to the appropriate therapeutic protocol.

The illustrative device 100 then enters a therapeutic mode of operation 176. During the therapeutic mode of operation, the device 100 illustratively produces a burst of positive pressure pulses from about 5 to about 40 cmH$_2$O combined with acoustic sound waves from about 5 to about 1200 Hz vibrating a column of gas in the airways. A sharp waveform of the air pulses is illustratively produced at a range from about 5 to about 100 Hz. The pressure amplitude starts low and is gradually increased according to patient tolerability considerations.

The illustrative device 100 finally enters a medication mode of operation 178. Following several cycles of therapeutic maneuvers during the therapeutic mode of operation 176 inducing secretion removal, the lungs are typically in an optimal condition to receive medication. As such, the device 100 efficiently delivers the aerosolized drug into the small-airways during the medication mode of operation 178.

As shown in FIG. 6B, the illustrative inlet air pressure at an air flow of 50-100 LPM may be about 15 cmH$_2$O to about 50 cmH$_2$O, wherein the inlet air pressure refers to the pressure provided by the air pump as described above. The illustrative outlet air pressure amplitude may be about 5 cmH$_2$O to about 50 cmH$_2$O, wherein the outlet air pressure amplitude refers to the air pressure delivered to the patient as part of the secretion clearance mechanism as described herein. The air pulse frequency may be about 5 Hz to about 700 Hz, wherein the air pulse frequency refers to the frequency generated by a flow pulsating element. In another illustrative embodiment, the device 100 may include more than one flow pulsating element which act in conjunction to generate the desired frequency response range. The outlet acoustical amplitude may be about 30 dB to about 105 dB, wherein the outlet acoustical amplitude refers to the acoustics delivered to the patient as part of the secretion clearance mechanism as discussed herein. The outlet acoustical pulse frequency, including overall frequencies resulting from harmonics and resonance, may be about 200 Hz to about 10,000 Hz, wherein the outlet acoustical pulse frequency refers to a range of frequencies allowing targeted clearance in different areas of the lungs based on the physical dimension of the airways. The outlet pressure during PEP maneuvers may be about 2 cmH$_2$O to about 25 cmH$_2$O, wherein the outlet pressure during PEP maneuvers refers to the back-pressure occurring during exhalation that ensures small airways do not collapse during the exhalation event.

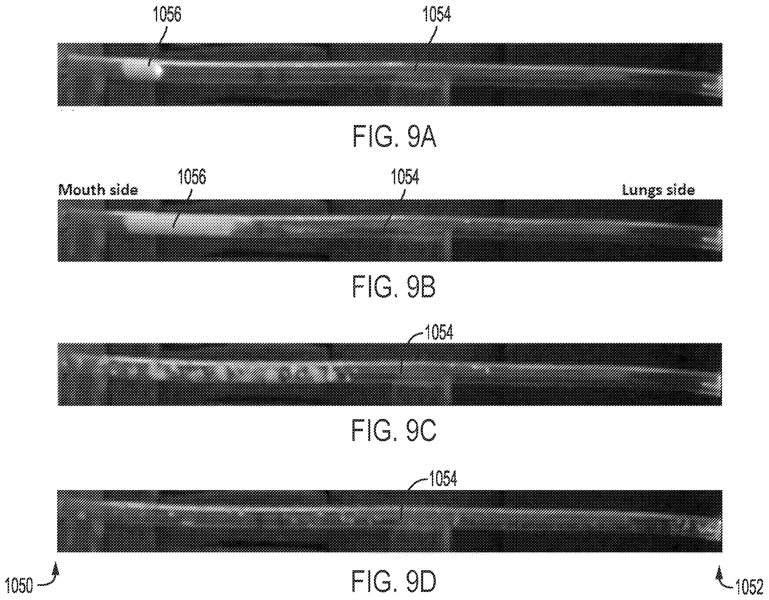

In a physiologically accurate branched airway of the human respiratory system, there is a need to generate acoustic waves at different frequencies commensurate with the different lengths of the different branches of subsequent generations of the airway. Referring to FIG. 9A, an exemplary branched airway is illustrated, although it is emphasized that geometry of airways differs between patients. Through a system of bifurcations, the consecutive airway tubes become smaller in diameter in length. In the first 16 generations of bifurcations, the typical diameter drops from about 1.8 cm to about 0.06 cm, although these dimensions may vary from person to person and between ages and sexes. Mucus may be located in any of these bifurcations. Since the resonance of a tube depends on the length of the tube, the diameter of the tube, and the distance between the mucus and the mouthpiece of the device, the optimal frequency of the air pulsations and the acoustic pulsations for effective mucus may vary. Further variations may occur depending on the physical properties of the mucus, which may depend on diseases, severity of mucus secretions, and temperature.

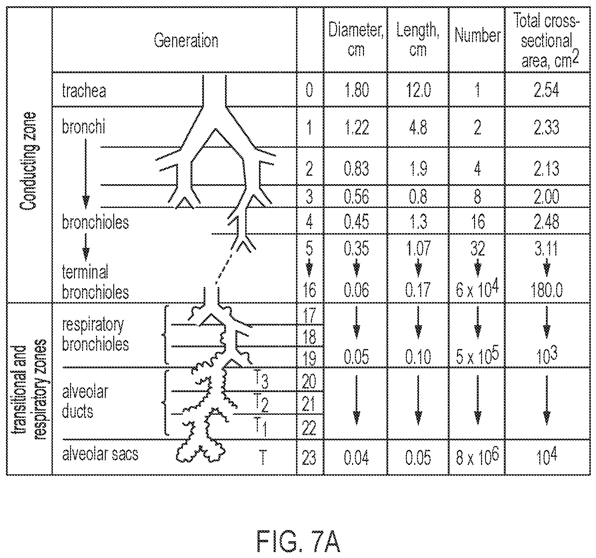

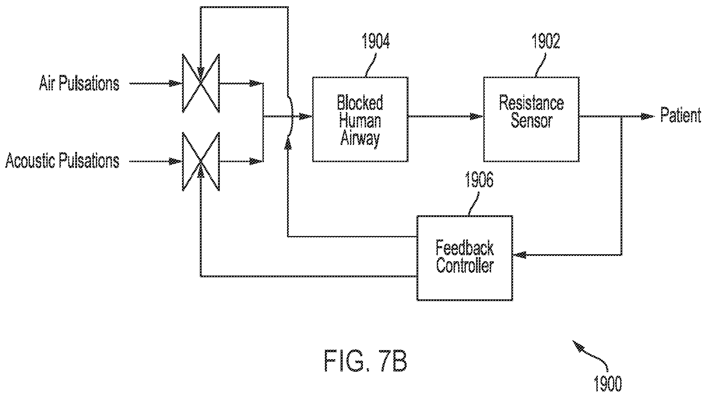

The illustrative devices described herein may overcome the varied needs of a patient in two ways: an open loop control and a closed loop control. In an open loop control system, the device will produce acoustic frequencies along a range of about 100 Hz to about 5000 Hz. (in addition to the other flow pulsations as detailed herein). These acoustic frequencies may be delivered as "white noise" within the given band-width or as a "chirp" in which a frequency sweep is delivered along this range of frequencies. In a closed loop control system, schematically illustrated by system 1900 in FIG. 7B, the device measures the pressure and flow resistance at the outlet of the device using resistance sensor 1902. As mucus is removed from a blocked airway 1904, resistance is reduced. A feedback controller 1906 determines the optimal air and acoustic pulsation frequency and amplitude to achieve minimum resistance using the measurements given by the resistance sensor 1902.

The resistance of the airway to airflow is defined by the equation below.

$$ R = \frac{\Delta P}{Q} $$

In the given equation, "R" denotes the resistance of the airway to airflow; "$\Delta P$" denotes the pressure required to drive the flow through the airway (in H2O or Pa), and "Q" denotes the flow rate (i.e. litres/min).

*Example 1*

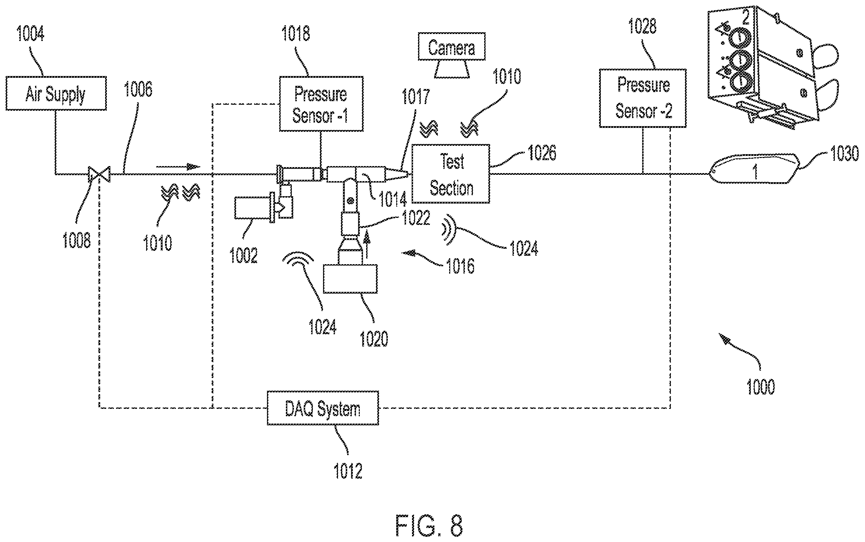

An exemplary test setup 1000 was prepared as illustrated in FIG. 8. Specifically, an intrapulmonary percussive ventilator (IPV) Phasitron 1002 was communicatively coupled to an air supply 1004 via tubing 1006. A solenoid valve 1008 was positioned along the tubing 1006 between the air supply 1004 and the IPV Phasitron 1002 to later (e.g., reduce or interrupt) the flow of the air from the air supply 1004 to provide a pulsed air supply 1010 to the IPV Phasitron 1002 as directed by a controller 1012 communicatively coupled to the solenoid valve 1008, wherein the controller 1012 was configured to control the flow rate, amplitude, and frequency of the pulsed air supply 1010 via the solenoid valve 1008. The IPV Phasitron 1002 was configured to allow the pulsed air supply 1010 to move through the IPV Phasitron 1002 during a simulated inhalation event and closed the passage during a simulated exhalation cycle, opening a different outlet for the pulsed air to exit the system before entering a T-junction 1016. The IPV Phasitron 1002 was coupled to an upper portion 1014 of the T-junction 1016, and a first pressure sensor 1018 was positioned substantially between the IPV Phasitron 1002 and the T-junction 1016. The pressure sensor 1018 was communicatively coupled to the controller 1012 to facilitate measurement and recordation of the air pressure as the pulsed air supply 1010 moved through the IPV Phasitron 1002, specifically measuring the pressure fluctuations at the inlet to the simulated airway.

A speaker or acoustic transducer 1020 was coupled to a base 1022 of the T-junction 1016 to provide acoustic oscillations 1024 through the base 1022 of the T-junction 1016 to meet the pulsed air supply 1010 at the upper portion 1014 of the T-junction 1016. The speaker 1020 was communicatively coupled to the controller 1012 to control the amplitude and frequency of acoustic oscillations 1024. The acoustic oscillations 1024 and the pulsed air supply 1010 exited the T-junction 1016 via a nozzle 1017 of the T-junction, wherein the nozzle 2017 transforms the acoustic oscillations to a pulsating train of vortices forming a synthetic jet as described above. The air then entered a test section 1026, which comprised additional tubing 1006, to mimic an esophagus, and simulated mucus as discussed further herein. A second pressure sensor 1028 was positioned downstream of the test section 1026 and communicatively coupled to the to measure and record the air pressure as it moved through the test section 1026 and the simulated mucus was removed. A simulated test lung 1030 was positioned at the end of the tubing 1006 opposite of the T-junction 1016 to complete the simulation of a respiratory system. During some testing sequences, the simulated test lung 1030 was a passive plastic lung. In other testing sequences, the simulated test lung 1030 was a mechanical lung that could either be turned on to simulate active breathing or turned off to simulate a passive lung to ascertain the impact of human breathing on the test setup.

Now referring to FIGS. 9A-9D, the target results for the application of the test setup 1000 is illustrated. For each of the steps (a)-(d) represented by FIGS. 9A-9D, respectively, a first end 1050 is closest to a simulated mouth, while a second end 1052 is closest to a simulated lung. Step (a) illustrates a simulated airway 1054 with a 2cm plug 1056 of simulated mucus, which fills the entire cross section of the simulated airway 1054. Step (b) illustrates penetration of the plug 1056, wherein the plug 1056 is punched open in the center via the combination of the pulsed air supply 1010 and the acoustic oscillations 1024.

Step (c) illustrates the breakdown of the simulated mucus immediately following the penetration illustrated in step (b). Specifically, the simulated mucus spread on the interior walls of the simulated airway 1054 and continues to be broken into small chunks by the continued pulsed air supply 1010 and the acoustic oscillations 1023. As the chunks are broken down, the smaller chunks are moved upstream (toward the mouth side), as shown in step (d), as a result of the pressure differential developed between the higher pressure end 1052 and the lower pressure end 1050.

Referring again to FIG. 8, the placement of the speaker 1020 allows the speaker to operate in a mode referred to as an acoustic jet, a synthetic jet, or a zero-net-mass jet. Synthetic jets are formed from the time harmonic motion of the acoustic oscillations 1024 created by the speaker 1020. During each simulated breathing cycle, the acoustic oscillations 1024 and the pulsed air supply 1010 are combined. As the combined air moves towards the nozzle 1017, a slug of air, or a synthetic jet, is ejected from the nozzle. Specifically, a vortex sheet is formed at the edge of the nozzle 1017, which rolls the vortex sheet into a vortex pair, or a vortex ring. At a high frequency, a train of vortex rings are formed resembling a pulsating continuous jet. The vortex ring travels through the tubing 1006 downstream under its own induced velocity, creating the synthetic jet. As the vortex ring moves away from the nozzle, and the combined air with it, a pressure drop occurs inside the T-junction 1016, allowing flow of air pressure to enter the T-junction 1016 and repeat the action. Formation of the synthetic jet requires a vibrating element, for example a speaker diaphragm, a piezoelectric element, a vibrating piston, and similar vibrational sources. A resonating volume, such as the acoustic pulsations, to produce a resonating chamber, and a housing with gradual constriction leading to a nozzle from which the train of vortices exit.

Illustratively, the acoustic vortices are at a much higher frequency than the air flow pulsations. Essentially, during each air flow pulsation, many vortices are generated and are carried downstream by the slower air flow pulsations. The mass of the slug that had exited the nozzle is equal to the air slug that enters upon the pressure drop, giving the synthetic jet their zero-net-mass-flux characteristic occurring due to the reversal of the flow. However, the zero-net-mass-flux characteristic of the synthetic jet does not translate into zero momentum flux. The flow pattern exiting the nozzle differs from the reverse flow. For example, while the exiting flow is unidirectional in the downstream direction, the reverse flow is entrained from all directions. As such, the synthetic jet has net momentum in the downstream direction and therefore behaves like a regular jet, despite being composed of a train of vortices. This composition results in the ability to penetrate further downstream with lower lateral spread. In other embodiments, the vibrating element of the synthetic jet may be based on mechanical means, such as a piston connected to a rotary motor; plasma; and piezoelectric means. The optimal vibration frequency is determined by the cavity volume and nozzle geometry as defined by the Helmholts resonator equation:

$$f = \frac{a}{2\pi} * \sqrt{\frac{A}{(V * Le)}}$$

In the above equation, a is the speed of sound, A is the nozzle area, V is the cavity volume, and Le = L+ 0.3D, where L is the nozzle's length and D is the nozzle's diameter. Further information related to acoustic jets, or synthetic jets, can be found in United States Patent Application Publication No. 2013/0186399, entitled "ACOUSTIC PRESSURE INDUCERS AND METHODS FOR TREATMENT OF OBSTRUCTIVE SLEEP APNEA" to Ephraim Gutmark, et al., filed on July 30, 2012.

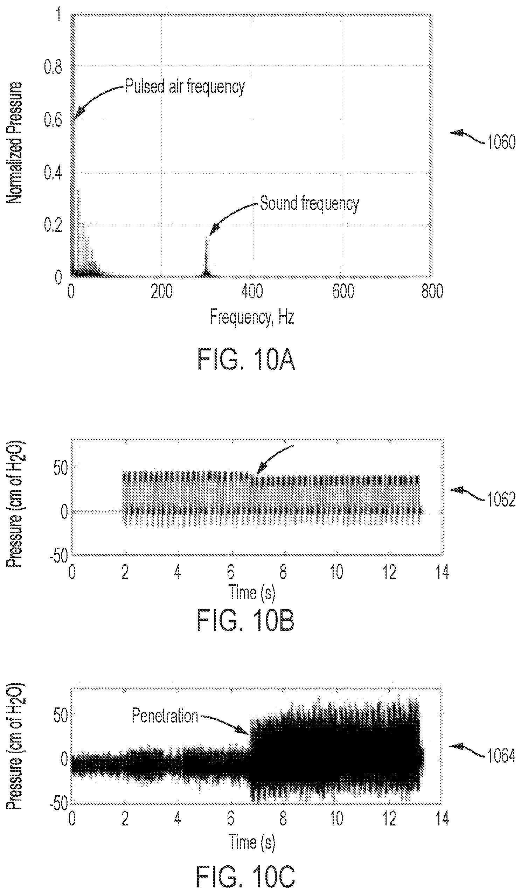

Graph 1060 of FIG. 10A clearly shows two distinct frequencies of applied air and sound pulsations as measured in the test setup 1000. The utilization of the two pressure sensors upstream 1018 and downstream 1028 of the test section 1026 makes it possible to measure the time at which the simulated mucus plug 1056 is penetrated. The pressure pulsations in graph 1062 of FIG. 10B show the measured normalized pressure during the testing period as measured by the upstream pressure sensor 1018, while the pressure pulsations in graph 1064 of FIG. 10C show the measured normalized pressure during the testing period as measured by the downstream pressure sensor 1028. The sudden increase in the magnitude of pressure fluctuations as measured by the downstream pressure sensor 1028 in graph 1062 indicates the moment of penetration. The difference between the starting time and the penetration time gives the time length of the penetration phase, which is about 7 seconds as shown. By changing and testing alternate supply air pressures, air pulsation frequencies, and sound frequencies, it is possible to derive parameters dictating penetration times across a wide range of test matrices. Generally, the method provided penetrates the simulated mucus plug 1056 within a few seconds. Graphs 1062 and 1064 also show that the pressure downstream after penetration is higher than the pressure upstream leading to the pressure differential that pushes the mucus outwards.

Generally, higher air supply pressures tend to reduce the time taken to penetrate the mucus plug 1056. At low flow pressures of 20 to 30 cmH$_2$O, the fastest penetration occurred at acoustic pulsations of about 350 Hz and low flow pulsations of about 200 beats per minute. As the air pressure was increased, the range of effective acoustic and flow pulsations was correspondingly increased. It should be noted that effective acoustic frequencies and air flow frequencies can change dependent upon parameters, such as patient's airway geometry (e.g., length and diameter), mucus type as related to the specific disease, acoustic pulsation source, etc.

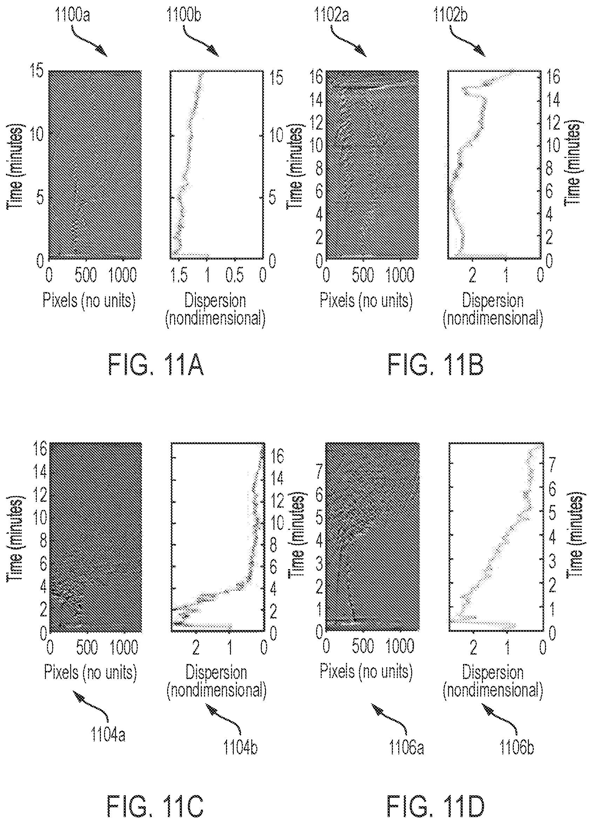

A custom image processing algorithm was used to decipher the breakdown, or dispersion, as well as the direction of movement, or transport, of the simulated mucus as shown in FIGS. 11A-11D. Graphs including an "a" reference provide an x-t diagram that shows the transport of the broken mucus within the testing tube. Movement of the pixels to the left indicates an upstream movement, or movement toward the mouth, while movement of the pixels to the right indicates a downstream movement, or movement toward the lungs. Graphs including a "b" reference provide an x-t diagram providing the amount of mucus in the tube as it changes from an initial value to a final level after a certain period of time. Complete clearance of the tube occurs as the value approaches zero.

Graphs 1100 of FIG. 11A illustrate the transport and dispersion properties of a test setup including air oscillations and active lung activity. Graphs 1102 of FIG. 11B illustrate the transport and dispersion properties of a test setup including acoustic oscillations and passive lung activity. Graphs 1104 of FIG. 11C illustrate the transport and dispersion properties of a test setup including acoustic oscillations and active lung activity. Graphs 1106 of FIG. 11D illustrate the transport and dispersion properties of a test setup including acoustic oscillations, air oscillations, and passive lung activity. As shown in FIGS. 11A-11D, the simulated mucus was only cleared from the simulated airway when the simulated airway was provided with both acoustic oscillations and air oscillations. The air oscillations may supplement the actions of an active lung or provided through solenoid valves without active breathing participation, or with passive lung activity.

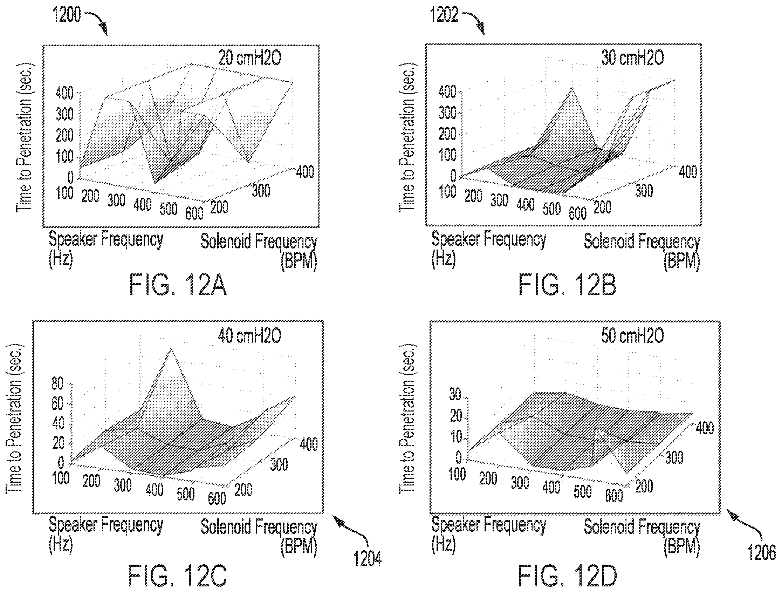

Additionally, FIGS. 12A-12D provides an illustration of the time to penetration of the simulated mucus plug based on the speaker frequency (Hz), the solenoid frequency (BPM), and air pressure (cmH$_2$O). Specifically, graph 1200 of FIG. 12A illustrates the time to penetration of a test setup with an air pressure of 20 cmH$_2$O, graph 1202 of FIG. 12B illustrates the time to penetration of a test setup with an air pressure of 30 cmH$_2$O, graph 1204 of FIG. 12C illustrates the time to penetration of a test setup with an air pressure of 40 cmH$_2$O, and graph 1206 of FIG. 12D illustrates the time to penetration of a test setup with an air pressure of 50 cmH$_2$O. As shown, a sound frequency of 490 Hz was most effective in providing penetration of the simulated mucus plug and additionally was the most effective in providing the upstream motion of the mucus simulant particles. The test data demonstrates that at low pressures (better for patients), the exact frequency combination of air and acoustic pulsations are more important. As the pressure increases, the range of effective frequency combinations widens.

During testing, the downstream pressure was about 60 cmH$_2$O higher than the upstream pressure in test setups including a single tube. The higher instantaneous unsteady pressure downstream of a simulated trachea is the mechanism that pushes the particles upstream. The pulsating air oscillations is acting as a carrier medium that penetrates the mucus, spreads the mucus, and mobilizes the broken mucus. The acoustic oscillations, via the synthetic jet mechanism discussed above, breaks up the spread mucus and establishes standing wave patterns within the airway, based on the branch length. The standing waves produce the pressure differential between a downstream section and an upstream section to drive the broken mucus upstream.

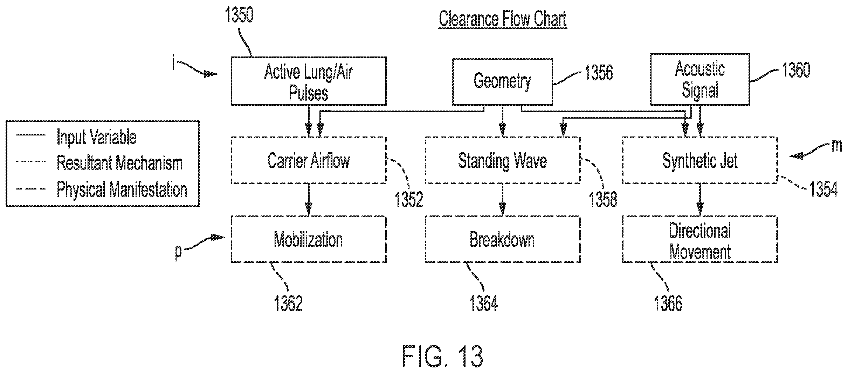

The clearance flow chart of FIG. 13 provides the dependency pathways between inputs ("i"), mechanisms ("m"), and processes ("p"). As shown, the use of an active lung and/or the given air oscillations 1350 result in carrier airflow 1352. The geometry 1356 of the used device, such as the T-junction described above including a nozzle, assists with creation of a standing wave 1358, facilitates carrier airflow 1352, and assists with creation of a synthetic jet 1354. The acoustic oscillations 1360 assist with creation of the standing wave 1358 and the synthetic jet 1354. The carrier airflow 1352 provides for mobilization 1362 of the simulated mucus particles. The standing wave 1358 provides for breakdown 1364 of simulated mucus plug. The synthetic jet 1354 provides for directional movement 1366 of the simulated mucus particles.

*Example 2*

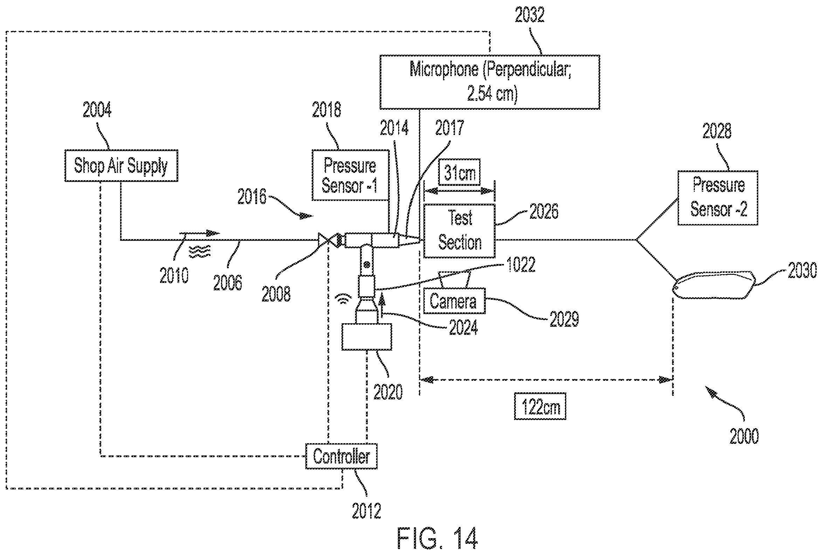

An exemplary test setup 2000 was prepared as illustrated in FIG. 14. An air supply 2004 was coupled to a T-junction 2016 with tubing 2006, wherein a 3/2 solenoid valve 2008 was placed between the air supply 2004 and the T-junction 2016 near the entry of the T-junction 2016 (instead of the IPV Phasitron as used above). The 3/2 solenoid valve 2008 operated to alter (e.g., reduce or interrupt) the flow of the air from the air supply 2004 to provide a pulsed air supply 2010 to the T-junction 2016 as directed by a controller 2012 communicatively coupled to the 3/2 valve 2008, wherein the controller 2012 was configured to control the flow rate, amplitude, and frequency of the pulsed air supply 2010 via the 3/2 solenoid valve 2008. The 3/2 solenoid valve 2008 was configured to allow the pulsed air supply 2010 to move through the 3/2 solenoid valve 2008 during a simulated inhalation event and closed the passage during a simulated exhalation cycle, opening a first outlet for the exhaled air to exit the device and a second outlet for the pulsed air to exit the device before entering T-junction 2016. A pressure sensor 2018 was communicatively coupled to the controller 2012 to facilitate measurement and recordation of the air pressure as the pulsed air supply 2010 moved through the 3/2 solenoid valve 2008, specifically measuring the pressure fluctuations at the inlet to the simulated airway discussed further herein.

A speaker 2020 was coupled to a base 2022 of the T-junction 2016 to provide acoustic oscillations 2024 through the base 2022 of the T-junction 2016 to meet the pulsed air supply 2010 at the upper portion 2014 of the T-junction 2016. The speaker 2020 was communicatively coupled to the controller 2012 to control the amplitude and frequency of acoustic oscillations 2024. The acoustic oscillations 2024 and the pulsed air supply 2010 combined within the T-junction 2016 and then exited the T-junction 2016 via a nozzle 2017 of the T-junction 2016, wherein the nozzle 2017 transforms the acoustic oscillations to a pulsating train of vortices forming a synthetic jet as described above. The air then entered a test section 2026, which comprised additional tubing 2006, to mimic an esophagus, and simulated mucus as discussed further herein. A microphone 2032 was positioned near the outlet of the nozzle 2017 (illustratively at a distance of 2.54 cm from the outlet of the nozzle 2017 and perpendicular thereto) and operatively coupled to the controller 2012 to measure and record the sound generated by the test setup 2000 during use.

A second pressure sensor 2028 was positioned downstream of the test section 2026 and communicatively coupled to the controller 2012 to measure and record the air pressure as it moved through the test section 2026 and the simulated mucus was removed. A camera 2029 may be directed toward the test section 2026 and configured to capture images of the simulated mucus. A simulated test lung 2030 (e.g., formed of a polymer) was positioned at the end of the tubing 2006 opposite of the T-junction 2016 to complete the simulation of a respiratory system. During some testing sequences, the simulated test lung 2030 was a passive plastic lung. In other testing sequences, the simulated test lung 2030 was a mechanical lung that could either be turned on to simulate active breathing or turned off to simulate a passive lung to ascertain the impact of human breathing on the test setup.

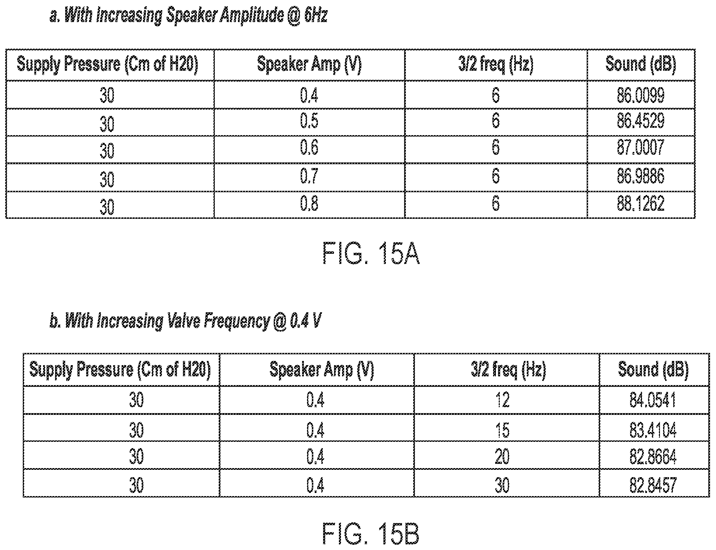

FIGS. 15A and 15B provides the sound recorded by the microphone 2032 of the test setup 2000 of FIG. 14 during use. Table "a" of FIG. 15A provides the recorded sound of the device in decibels, wherein the air supply pressure remained at 30 cmH2O, the frequency of the 3/2 solenoid valve frequency remained at 6 Hz, and the speaker amplitude increased. At a speaker amplitude of 0.4 V, the recorded sound decibel value was 86.0099 dB. At a speaker amplitude of 0.5 V, the recorded sound decibel value was 86.4529 dB. At a speaker amplitude of 0.6 V, the recorded sound decibel value was 87.0007 dB. At a speaker amplitude of 0.7 V, the recorded sound decibel value was 86.9886 dB. At a speaker amplitude of 0.8 V, the recorded sound decibel value was 88.1262 dB.

Table "b" of FIG. 15B provides the recorded sound of the device in decibels, wherein the air supply pressure remained at 30 cmH2O, the speaker amplitude remained at 0.4 V, and the 3/2 solenoid valve frequency increased. At a 3/2 solenoid valve frequency of 12 Hz, the recorded sound decibel value was 84.0541 dB. At a 3/2 solenoid valve frequency of 15 Hz, the recorded sound decibel value was 83.4104 dB. At a 3/2 solenoid valve frequency of 20 Hz, the recorded sound decibel value was 82.8664 dB. At a 3/2 solenoid valve frequency of 30 Hz, the recorded sound decibel value was 82.8457 dB. As shown, as the 3/2 solenoid valve frequency increases, the sound produced by the device decreases.

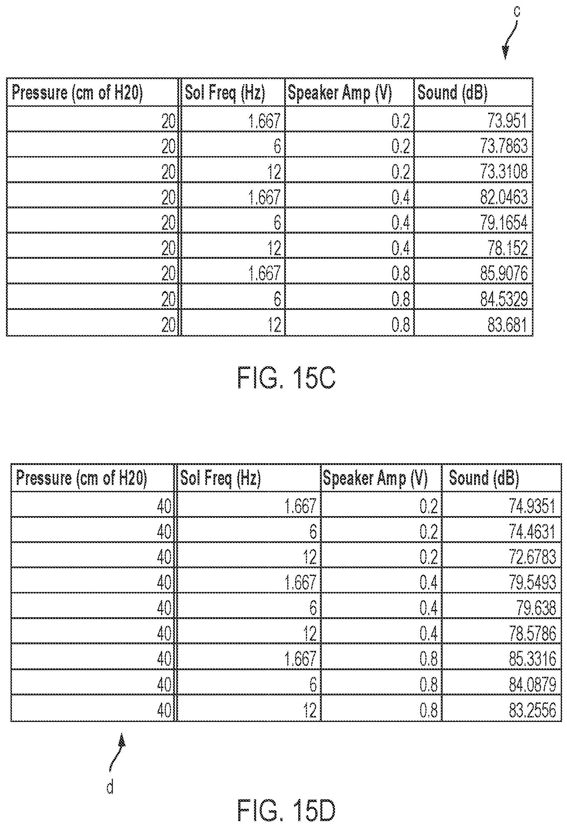

FIGS. 15C and 15D provides further information related to the sound recorded by the microphone 2032 of the test setup 2000 of FIG. 14 during use. Table "c" of FIG. 15C provides the recorded sound of the device in decibels, wherein the air supply pressure remained at 20 cmH2O over varied 3/2 solenoid valve frequencies and varied speaker amplitudes. At a 3/2 solenoid valve frequency of 1.667 Hz and a speaker amplitude of 0.2 V, the recorded sound decibel value was 73.951 dB. At a 3/2 solenoid valve frequency of 6 Hz and a speaker amplitude of 0.2 V, the recorded sound decibel value was 73.7863 dB. At a 3/2 solenoid valve frequency of 12 Hz and a speaker amplitude of 0.2 V, the recorded sound decibel value was 73.3108 dB.

Still referring to table "c" of FIG. 15C, at a 3/2 solenoid valve frequency of 1.667 Hz and a speaker amplitude of 0.4 V, the recorded sound decibel value was 82.0463 dB. At a 3/2 solenoid valve frequency of 6 Hz and a speaker amplitude of 0.4 V, the recorded sound decibel value was 79.1654 dB. At a 3/2 solenoid valve frequency of 12 Hz and a speaker amplitude of 0.4 V, the recorded sound decibel value was 78.152 dB. At a 3/2 solenoid valve frequency of 1.667 Hz and a speaker amplitude of 0.8 V, the recorded sound decibel value was 85.9076 dB. At a 3/2 solenoid valve frequency of 6 Hz and a speaker amplitude of 0.8 V, the recorded sound decibel value was 84.5329 dB. At a 3/2 solenoid valve frequency of 12 Hz and a speaker amplitude of 0.8 V, the recorded sound decibel value was 83.681 dB.

Referring to table "d" of FIG. 15D, the recorded sound of the device in decibels is provided, wherein the air supply pressure remained at 40 cmH2O over varied 3/2 solenoid valve frequencies and varied speaker amplitudes. At a 3/2 solenoid valve frequency of 1.667 Hz and a speaker amplitude of 0.2 V, the recorded sound decibel value was 74.9351 dB. At a 3/2 solenoid valve frequency of 6 Hz and a speaker amplitude of 0.2 V, the recorded sound decibel value was 74.4631 dB. At a 3/2 solenoid valve frequency of 12 Hz and a speaker amplitude of 0.2 V, the recorded sound decibel value was 72.6783 dB.

Still referring to table "d" of FIG. 15D, at a 3/2 solenoid valve frequency of 1.667 Hz and a speaker amplitude of 0.4 V, the recorded sound decibel value was 79.5493 dB. At a 3/2 solenoid valve frequency of 6 Hz and a speaker amplitude of 0.4 V, the recorded sound decibel value was 79.638 dB. At a 3/2 solenoid valve frequency of 12 Hz and a speaker amplitude of 0.4 V, the recorded sound decibel value was 78.5786 dB. At a 3/2 solenoid valve frequency of 1.667 Hz and a speaker amplitude of 0.8 V, the recorded sound decibel value was 85.3316 dB. At a 3/2 solenoid valve frequency of 6 Hz and a speaker amplitude of 0.8 V, the recorded sound decibel value was 84.0879 dB. At a 3/2 solenoid valve frequency of 12 Hz and a speaker amplitude of 0.8 V, the recorded sound decibel value was 83.2556 dB.

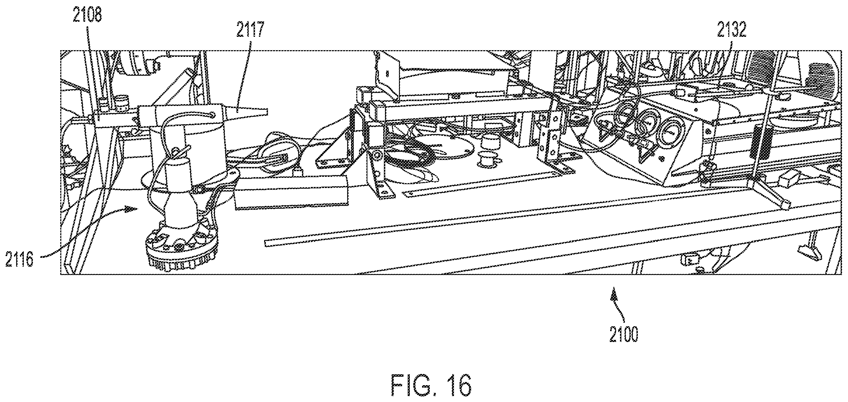

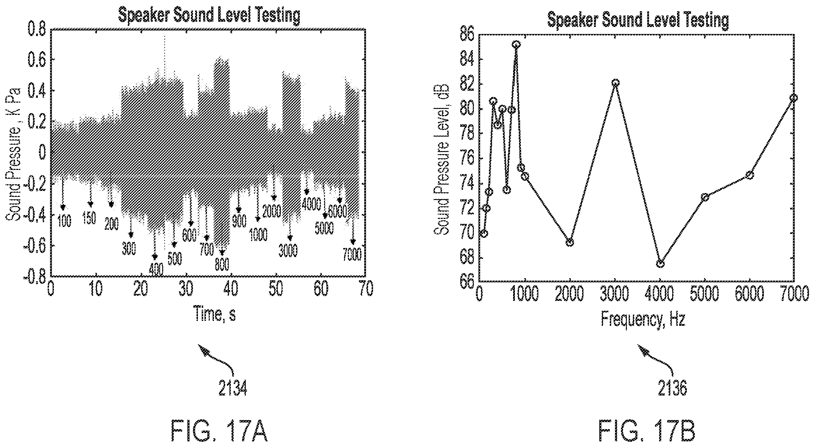

A test setup 2100 shown in FIG. 16 is similar to the test setup 2000 shown in FIG. 16, however, in the test setup 2100, the microphone 2132 is positioned 1 meter away from the nozzle 2117 of the T-junction 2116. The 3/2 solenoid valve 2108 was operated at a range of 100 Hz to 7000 Hz, and the sound was measured and recorded using the microphone 2132. The sound pressure produced over the tested frequencies over 70 seconds is provided in graph 2134 of FIG. 17A, while an average sound of 78 dB was recorded as shown by graph 2136 of FIG. 17B.

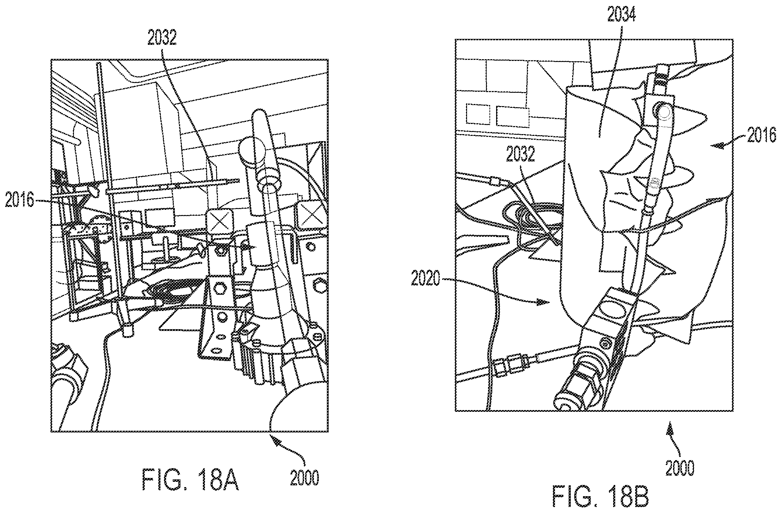

FIG. 18A shows the test setup 2000, wherein the microphone 2032 is positioned at the exit of the nozzle 2017 (FIG. 14). FIG. 18B shows the test setup 2000 with the addition of foam 2034 wrapped around the T-junction 2016, wherein the microphone 2032 is positioned near the speaker 2020. Each test setup 2000 was operated, with the sound output measured and recorded as described further herein.

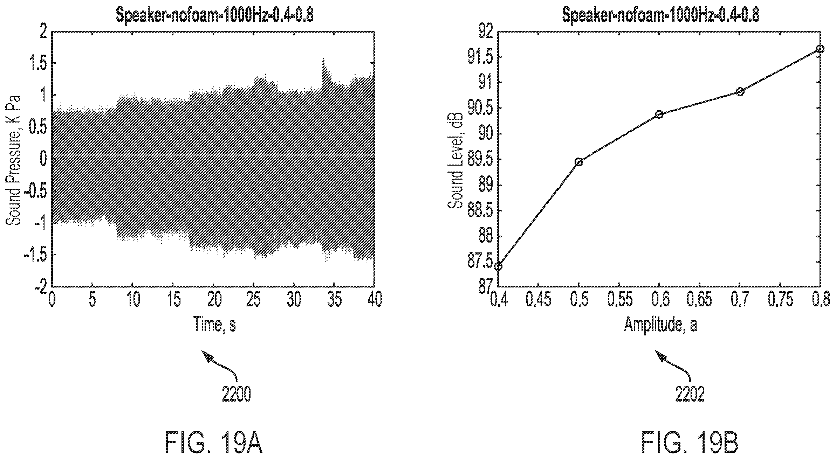

FIGS. 19A and 19B provide the results for the test setup of FIG. 18A, wherein the microphone was positioned at the exit of the nozzle and test setup did not include a foam surrounding. Graph 2200 of FIG. 19A shows the varying sound pressure (Pa) over 40 seconds, wherein the 3/2 solenoid valve was operated at a frequency of 1000 Hz, and the speaker was operated at an amplitude of 0.4 V to 0.8 V as further portrayed in graph 2202 of FIG. 19B. Graph 2202 illustrates the change in sound level as the speaker amplitude fluctuates according to graph 2200. As shown, as the speaker amplitude increased, the sound level also increased. At a speaker amplitude of 0.4 V, the microphone measured a sound level of about 87.4 dB; at a speaker amplitude of 0.5 V, the microphone measured a sound level of about 89.4 dB; at a speaker amplitude of 0.6 V, the microphone measured a sound level of about 90.4 V; at a speaker amplitude of 0.7 V, the microphone measured a sound level of about 90.8 V; and at a speaker amplitude of 0.8 V, the microphone measured a sound level of about 91.6 dB.

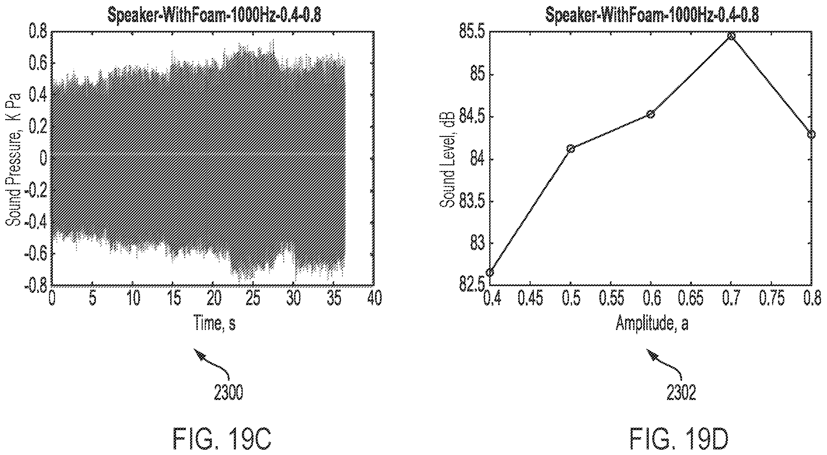

FIGS. 19C and 19D provide the results for the test setup of FIG. 18A, wherein the microphone was positioned at the exit of the nozzle, however, the test setup included a foam surrounding. Graph 2300 of FIG. 19C shows the varying sound pressure (Pa) over about 37 seconds, wherein the 3/2 solenoid valve was operated at a frequency of 1000 Hz, and the speaker was operated at an amplitude of 0.4 V to 0.8 V as further portrayed in graph 2302 of FIG. 19D. As shown, as the speaker amplitude increased, the sound level also generally increased until the maximum amplitude was reached. At a speaker amplitude of 0.4 V, the microphone measured a sound level of about 82.7 dB; at a speaker amplitude of 0.5 V, the microphone measured a sound level of about 84.2 dB; at a speaker amplitude of 0.6 V, the microphone measured a sound level of about 84.5 dB; at a speaker amplitude of 0.7 V, the microphone measured a sound level of about 85.5 dB; and at a speaker amplitude of 0.8 V, the microphone measured a sound level of about 84.3 dB. Comparing FIGS. 19A, 19B with FIGS. 19C, 19D, the foam significantly lowered the sound level of the test setup.

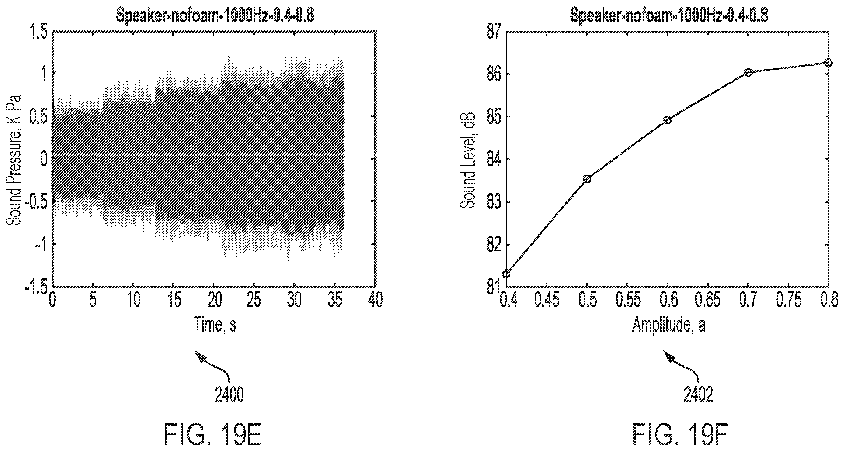

FIGS. 19E and 19F provide the results for the test setup of FIG. 18B, wherein the microphone was positioned near the speaker; however, the test setup did not include a foam surrounding. Graph 2400 of FIG. 19E shows the varying sound pressure (Pa) over about 37 seconds, wherein the 3/2 solenoid valve was operated at a frequency of 1000 Hz, and the speaker was operated at an amplitude of 0.4 V to 0.8 V as further portrayed in graph 2402 of FIG. 19F. As shown, as the speaker amplitude increased, the sound level also increased. At a speaker amplitude of 0.4 V, the microphone measured a sound level of about 81.3 dB; at a speaker amplitude of 0.5 V, the microphone measured a sound level of about 83.5 dB; at a speaker amplitude of 0.6 V, the microphone measured a sound level of about 84.9 dB; at a speaker amplitude of 0.7 V, the microphone measured a sound level of about 86 dB; and at a speaker amplitude of 0.8 V, the microphone measured a sound level of about 86.2 dB.

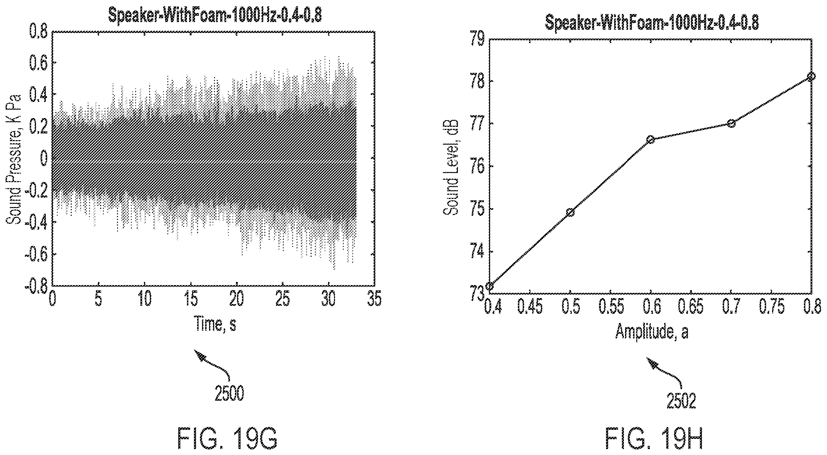

FIGS. 19G and 19H provide the results for the test setup of FIG. 18B, wherein the microphone was positioned near the speaker and the test setup included a foam surrounding. Graph 2500 of FIG. 19G shows the varying sound pressure (kPa) over about 37 seconds, wherein the 3/2 solenoid valve was operated at a frequency of 1000 Hz, and the speaker was operated at an amplitude of 0.4 V to 0.8 V as further portrayed in graph 2502 of FIG. 19H. As shown, as the speaker amplitude increased, the sound level also increased. At a speaker amplitude of 0.4 V, the microphone measured a sound level of about 73.2 dB; at a speaker amplitude of 0.5 V, the microphone measured a sound level of about 74.9 dB; at a speaker amplitude of 0.6 V, the microphone measured a sound level of about 76.6 dB; at a speaker amplitude of 0.7 V, the microphone measured a sound level of about 77 dB; and at a speaker amplitude of 0.8 V, the microphone measured a sound level of about 78.1 dB. Comparing FIGS. 19E, 19F with FIGS. 19G, 19H, the foam significantly lowered the sound level of the test setup.

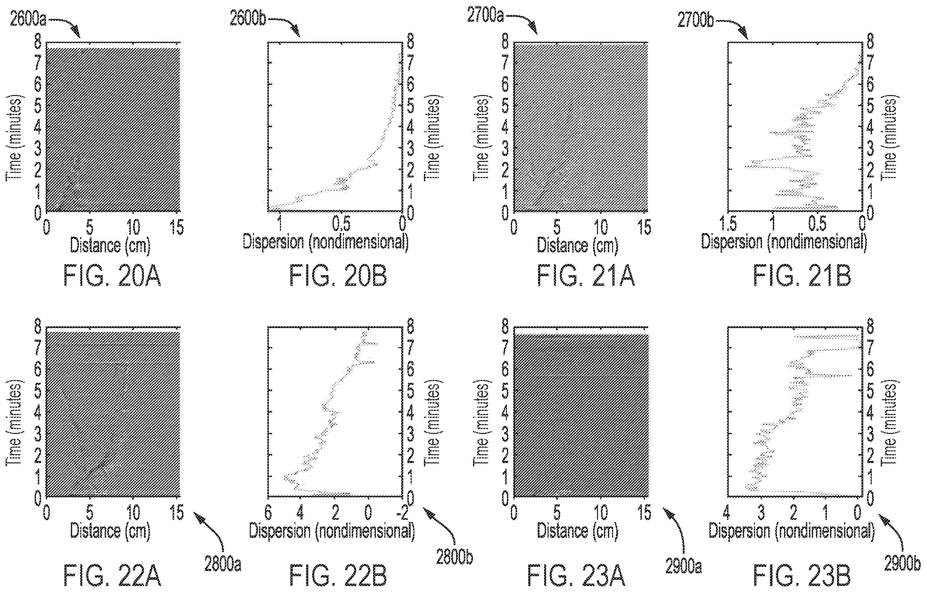

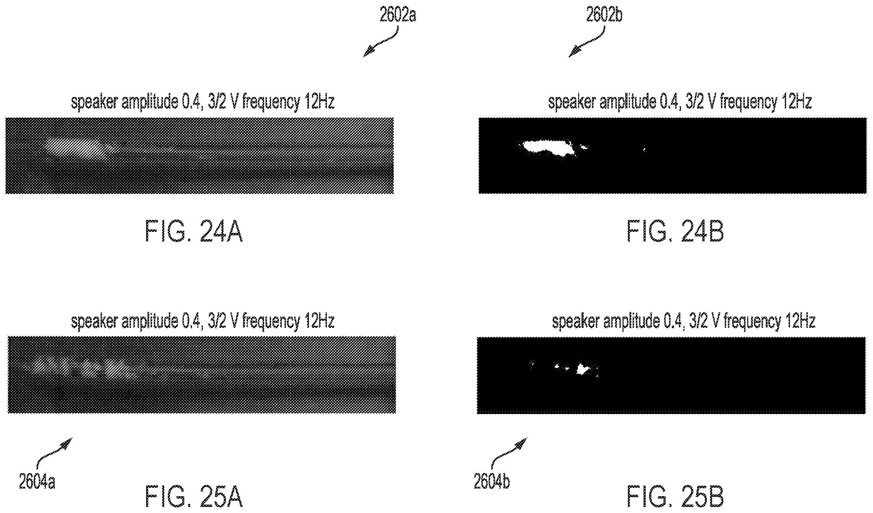

Now referring to FIGS. 20A-23B, the movement of mucus simulant, which may have similar characteristics as mayonnaise, is provided as moved through test setup 2000 of FIG. 14. Graph 2600a of FIG. 20A shows the movement in centimeters of mucus simulant over eight minutes at a 3/2 solenoid valve frequency of 12 Hz. Graph 2600b of FIG. 20B shows the dispersion of the mucus simulant during the same test period. FIGS. 24A and 24B show the movement of the mucus simulant according to graphs 2600a and 2600b of FIGS. 20A and 20B. More particularly, graphs 2602a and 2602b of FIGS. 24A and 24B show the position of the mucus simulant before the test, and graphs 2604a and 2604b of FIGS. 25A and 25B show the position and dispersion of the mucus simulant at the end of the eight minutes.

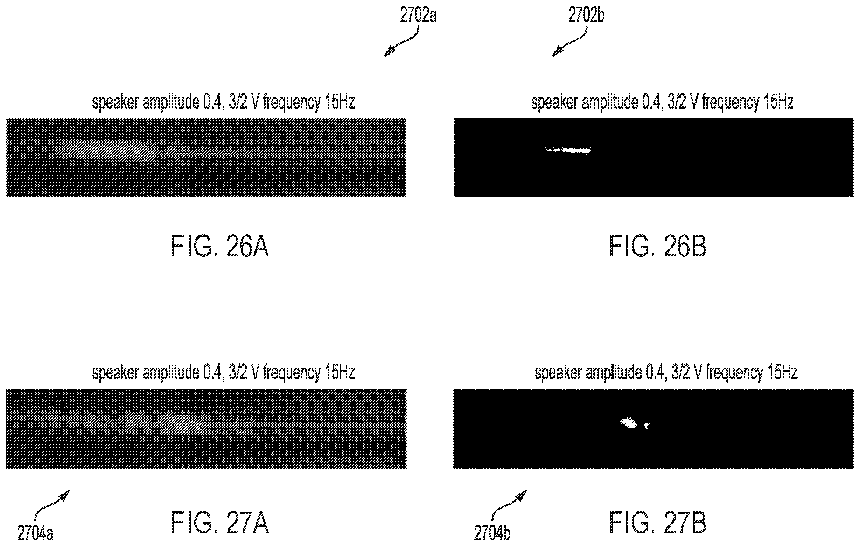

Referring further to FIG. 21A, graph 2700a shows the movement in centimeters of mucus simulant over eight minutes at a 3/2 solenoid valve frequency of 15 Hz. Graph 2700b of FIG. 21B shows the dispersion of the mucus simulant during the same test period. FIGS. 26A and 26B show the movement of the mucus simulant according to graphs 2700a and 2700b of FIGS. 21A and 21B. More particularly, graphs 2702a and 2702b of FIGS. 26A and 26B show the position of the mucus simulant before the test, and graphs 2704a and 2704b of FIGS. 27A and 27B show the position and dispersion of the mucus simulant at the end of the eight minutes.

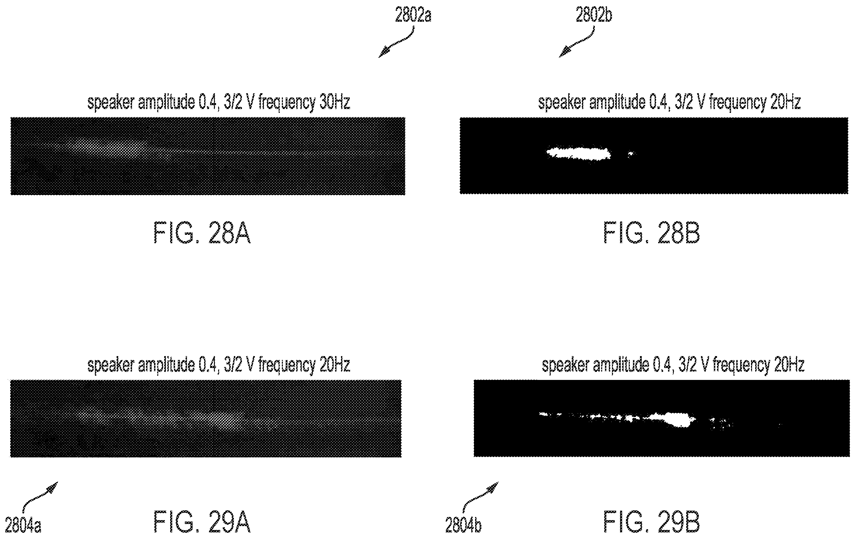

Referring further to FIG. 22A, graph 2800a shows the movement in centimeters of mucus simulant over eight minutes at a 3/2 solenoid valve frequency of 20 Hz. Graph 2800b of FIG. 22B shows the dispersion of the mucus simulant during the same test period. FIGS. 28A and 28B show the movement of the mucus simulant according to graphs 2800a and 2800b of FIGS. 22A and 22B. More particularly, graphs 2802a and 2802b of FIGS. 28A and 28B show the position of the mucus simulant before the test, and graphs 2804a and 2804b of FIGS. 29A and 29B show the position and dispersion of the mucus simulant at the end of the eight minutes.

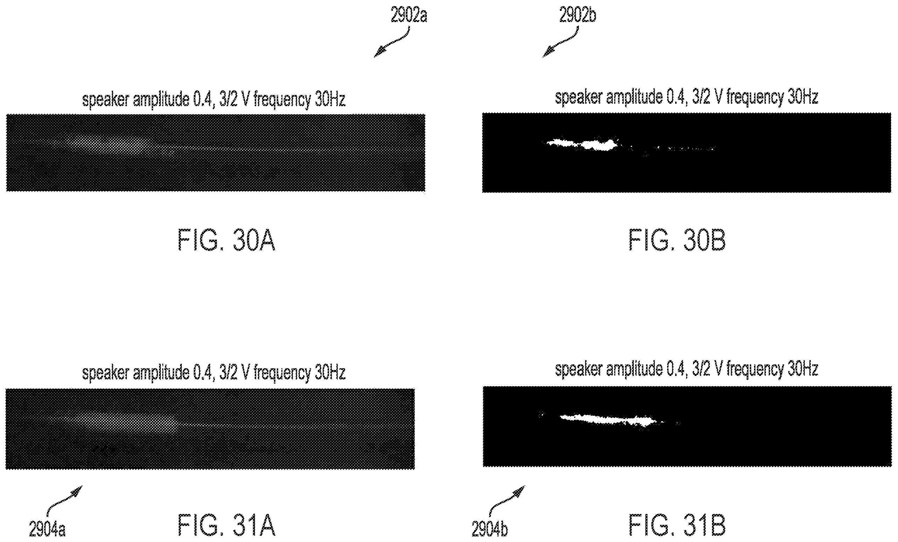

Returning further to FIG. 23A, graph 2900a shows the movement in centimeters of mucus simulant over eight minutes at a 3/2 solenoid valve frequency of 30 Hz. Graph 2900b of FIG. 23B shows the dispersion of the mucus simulant during the same test period. FIG. 30A and 30B show the movement of the mucus simulant according to graphs 2900a and 2900b of FIGS. 23A and 23B. More particularly, graphs 2902a and 2902b of FIGS. 30A and 30B show the position of the mucus simulant before the test, and graphs 2904a and 2904b of FIGS. 31A and 31B show the position and dispersion of the mucus simulant at the end of the eight minutes.

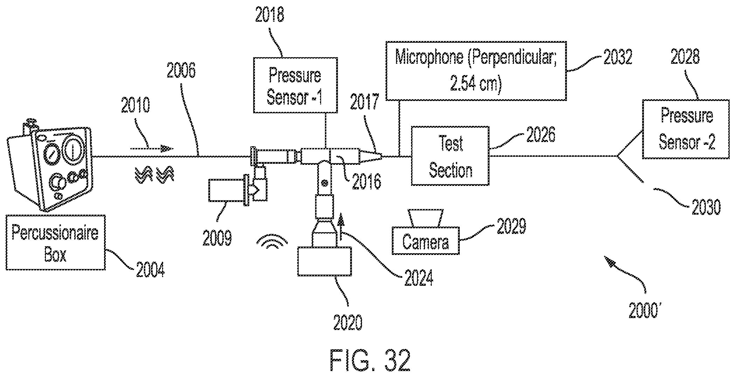

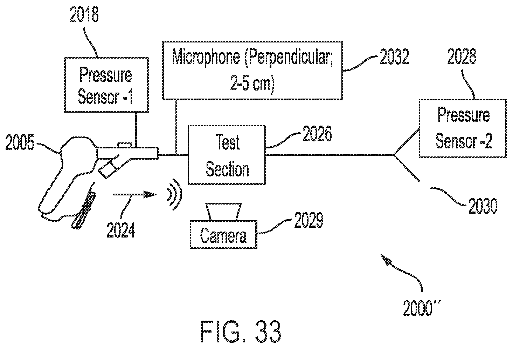

FIGS. 14, 32 and 33 illustrative different test setups 2000, 2000' and 2000" for predicate device comparison tests. Illustratively, an intrapulmonary percussive ventilation (IPV) device available from Percussionaire of Sandpoint, Idaho was used in the test setup 2000' of FIG. 32, and an acoustical percussor such as a Vibralung device available from WestMed of Tuscon, Arizona was used in the test setup 2000" of FIG. 33.

FIG. 32 is a schematic view, similar to FIG. 14, of an illustrative test setup 2000' for testing the effects of oscillating air pressure pulses and acoustic oscillations on a simulated airway. It should be appreciated that like reference numbers in FIGS. 14 and 32 identify similar components. The test setup 2000' includes a Percussionaire® IPV device 2004 supplying air to an IPV Phasitron® 2009. During testing, the IPV device 2004 was operated per manufacturer guidelines. The IPV device 2004 was tested at pressure settings ranging from 20 cnH$_2$O to 50 cmH$_2$O at Low, Mid and High settings per the user manual.

FIG. 33 is a schematic view, similar to FIG. 32, of an illustrative test setup for testing the effects of acoustic oscillations on a simulated airway. It should be appreciated that like reference numbers in FIGS. 14 and 33 identify similar components. The test setup 2000" includes a Vibralung acoutstical percussor 2005. During testing, the percussor 2005 was operated per manufacturer guidelines at Low, Mid, High, and Random Noise (R2 and R5) settings.

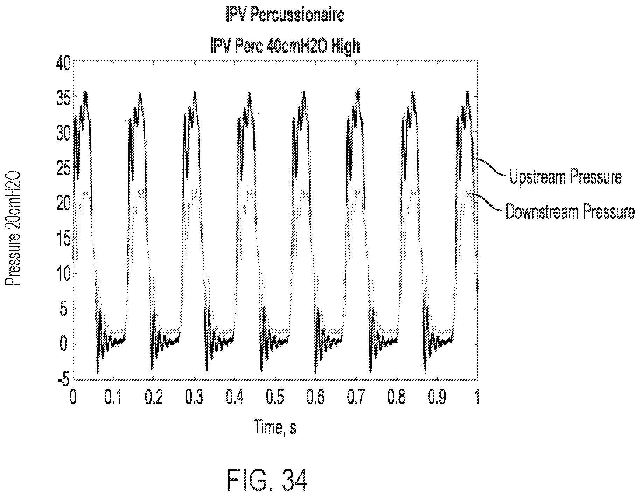

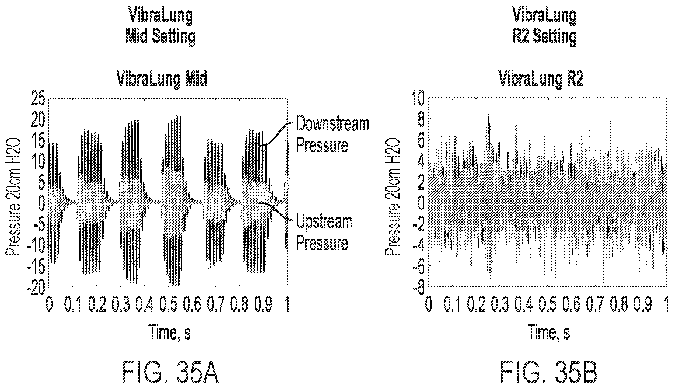

FIG. 34 is a graph illustrating recorded pressures (20cmH2O) at the first, upstream pressure sensor 2018 and the second, downstream pressure sensor 2028 over time for the illustrative test setup 2000' of FIG. 32. FIGS. 35A and 35B are graphs illustrating recorded pressures (20cmH2O) at the first, upstream pressure sensor 2018 and the second, downstream pressure sensor 2028 over time for the illustrative test setup 2000" of FIG. 33. As seen from these results, the IPV device 2004 and the percussor 2005 result in a higher pressure upstream compared to the downstream pressure.

Testing of the test setups 2000' and 2000" continued by adding a mucus simulant to the respective test sections 2026, and connecting the passive lung 2030 on the distal end (thereby simulating a full blockage of the airway with no respiratory support from the air flow that occurs during normal respiration). The test sections 2026 of the test setups 2000, 2000' and 2000" were illustratively plastic tubes having a length of approximately 14 inches and an internal diameter of approximately 0.250 inches. In the test setup 2000' of FIG. 32, the IPV device 2004 pushed the mucus plug slightly deeper into the test section 2026 - towards the passive lung 2030 and no penetration or clearance was observed after the recommended operation duration. With the test setup 2000" including the percussor 2005, the mucus plug was penetrated and the mucus spread downstream and coated the walls of the test section 2026. However, after this initial penetration, no clearance of the mucus was observed after the recommended operation duration.

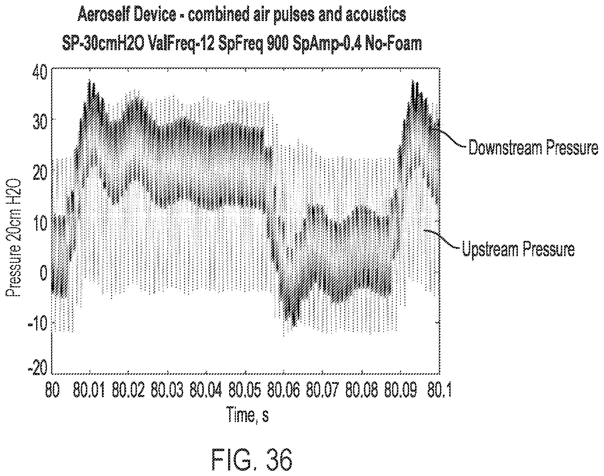

Pressure trace results from operation of the test setup 2000 of FIG. 14 are shown in FIG. 36. More particularly, FIG. 36 is a graph illustrating recorded pressures (20cmH2O) at the first, upstream pressure sensor 2018 and the second, downstream pressure sensor 2028 over time for the illustrative test setup 2000. As clearly seen, the combination of air pulses and acoustics, and with the resultant synthetic jet, creates a higher pressure downstream when compared to upstream. When a mucus simulant was added to the test section 2026, a rapid penetration followed by complete clearance was observed using the test setup 2000. These results clearly highlight the benefit of the test setup 2000 of the present disclosure over conventional devices. The ability to self-mobilize mucus can potentially allow for the test setup 2000 to assist airway clearance of patients with weakened or no cough function such as patients with COPD, patients with neuromuscular diseases such as muscular dystrophy (Duchenne), and patients with spinal diseases such as spinal muscular atrophy to name a few.

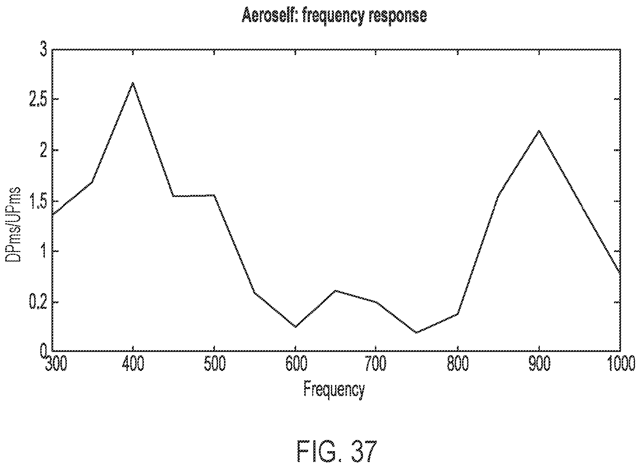

FIG. 37 is a graph illustrating frequency response of the illustrative test setup 2000 of FIG. 14. The results in FIG. 37 show delta P rms (root mean square value of pressure difference between the downstream and upstream pressures) values for the test setup 2000 as a function of the applied acoustic frequency. As seen from the results in FIG. 37, the test setup 2000 can be "tuned" for a specific section of the airway to maximize the pressure difference and assist with mucus clearance from the airways. This includes the ability to "tune" the system to target different sections of the lungs.

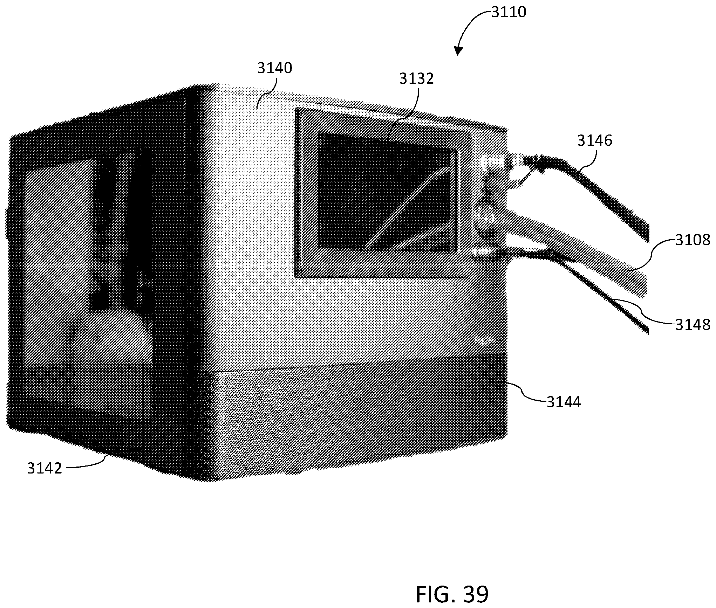

FIGS. 39-43 show another illustrative device of the present disclosure including many of the features detailed above. As such, in the following description, like reference numbers are used to identify similar components.

The illustrative airway clearance device 3100 is configured to be a portable unit illustratively for home use by patients with pulmonary diseases. The device 3100 illustratively includes many components similar to those detailed above in connection with the device 100 of FIGS. 1A-2C. The illustrative device 3100 includes a hand unit 3102, a base unit 3110, and a tube 3108 coupling the base unit 3110 and the hand unit 3102. A conventional fluid coupling 3109 illustratively connects the tube 3108 to the base unit 3110.

The base unit 3110 illustratively includes an air flow generator or air pump 3112 (illustratively a Micronel U-71HX blower) that draws air from the atmosphere via a high-efficiency filter 3117. Operation of the blower 3112 (and all components of the illustrative device 3100) is controlled via a controller 3114, illustratively an Intel i5-9600K processor. The air output of the blower 3112 passes via a first pressure sensor 3122 and a first flow sensor 3126 to an air pulsation unit 3116, illustratively a valve sub-assembly.

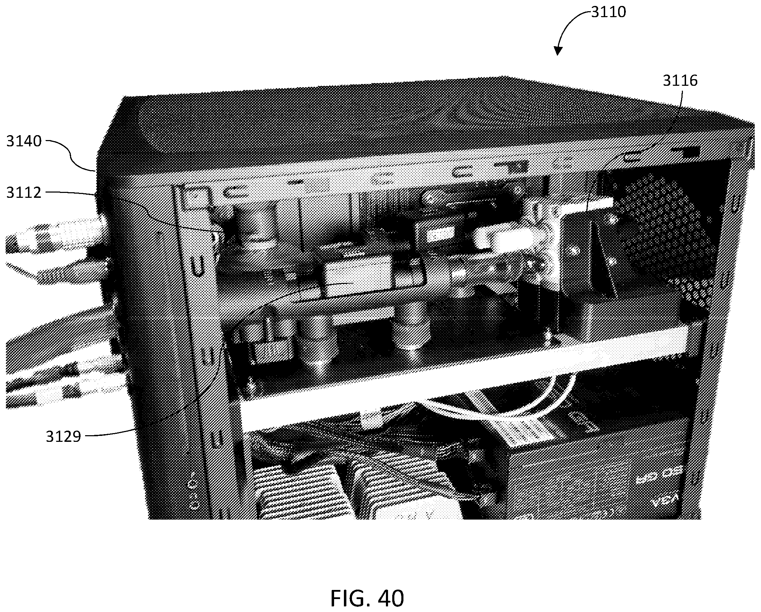

Operation of the blower 3112 is configured to be programmable for either a desired pressure level (i.e., a constant pressure mode) or a desired flow rate (i.e., a constant flow mode). In the constant pressure mode, the signal from first pressure sensor 3122, which measures differential pressure against ambient pressure, is used to ensure that the air blower 3112 is providing the desired pressure with respect to ambient pressure. The illustrative air pulsation unit 3116 consists of a pair of electrically operable valves (e.g., high-speed solenoid valves) 3130a and 3130b mounted in parallel as an assembly. Illustratively, the solenoid valves 3130a, 3130b may be Model No. MHE4-MS51H-3/2G-QS-8-K available from Festo Corporation of Islandia, New York USA. The air flow from the blower 3112 is bifurcated at a conventional upstream fitting 3118a to the parallel solenoid valves 3130a and 3130b, and the air flow output of the valves 3130a and 3130b is merged to a common air flow output line at a conventional downstream fitting 3118b. The use of the two valves 3130a and 3130b in parallel allows for a desired flow rate. The output of the valve sub-assembly 3116 is delivered in the form of constant frequency (or sweep of frequencies as further detailed herein) air-pulses to the hand unit 3102 with additional pressure/flow sensors 3124 and 3128 to monitor the output from the valve sub-assembly 3116. With reference to FIG. 40, the first pressure sensor 3122 and the first flow sensor 3126, and/or the second pressure sensor 3124 and the second flow sensor 3128 may define a pressure/flow sensor assembly 3129 (FIG. 40).

The illustrative base unit 3110 further includes peripheral components such as power supplies, data acquisition boards, signal amplifier for speakers, a user interface (such as a touch screen display) 3132, and mechanical and electrical interface ports (such as a USB connector 3134). The touch screen display 3132 is operably coupled to the controller 3114 and allows the user to enter desired operational parameters, such as air pressure, air-pulse frequency, flow rate, acoustic amplitude, and acoustic pulse frequency within the ranges detailed below. The touch screen display 3132 also displays the operational status such as elapsed time, pressure magnitude, air-pulse, sound frequency and its FFT transform from a user-selected sensor set (where a sensor set includes a pressure sensor and a flow sensor).

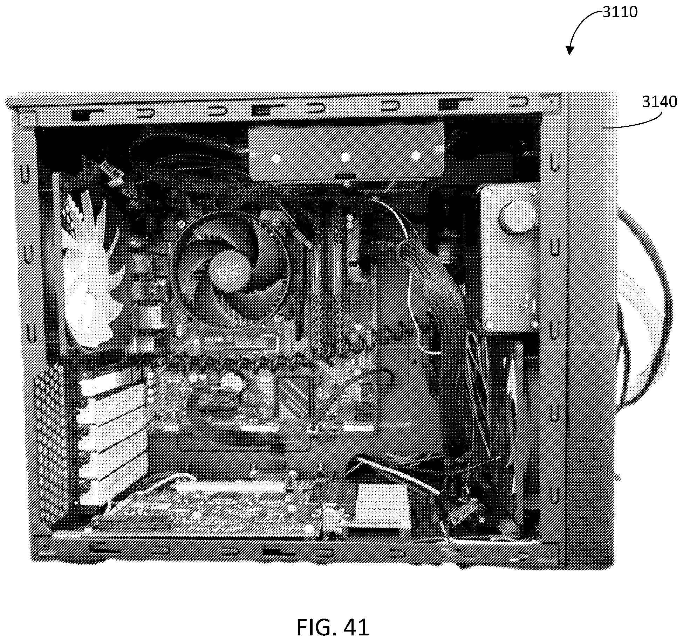

With reference now to FIGS. 39-41, the base unit 3110 illustratively includes a housing 3140 supporting the touch screen display 3132. Left and right side covers 3142 and 3144 provide access to the internal components received within the housing 3140. The covers 3142 and 3144 are shown removed in FIGS. 40 and 41, respectively, for clarity. Signal cables 3146 may be operably coupled between the controller 3114 of the base unit 3110 and the hand unit 3102. Similarly, speaker, power and control signal cables 3148 may be operably coupled between the controller 3114 of the base unit 3110 and the hand unit 3102.

Illustrative operational ranges of the device 3100 include:

- Air Pressure amplitude: 4-50 cm H2O

- Air pulse frequency: 3 - 125 Hz

- Flow rate: 50-100 liter per minute (LPM)

- Acoustic amplitude: Average of 80 dB measured 1 meter from the outlet of hand unit 3102.

- Acoustic pulse Frequency: 200- 7000 Hz

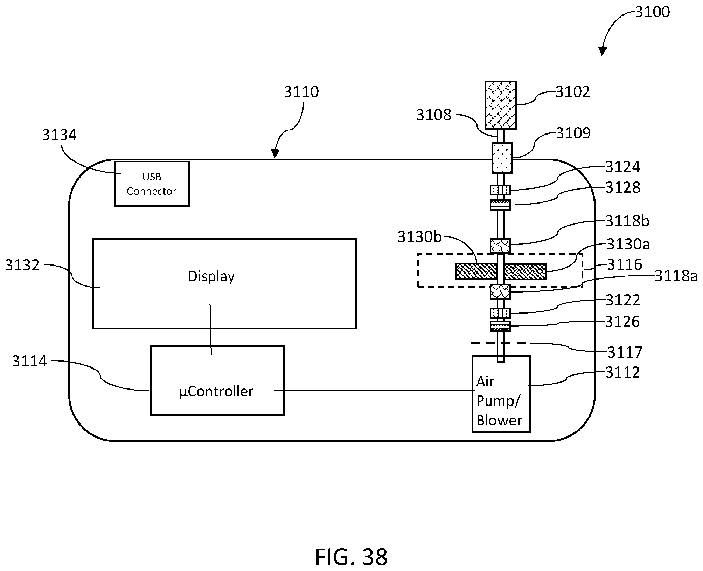

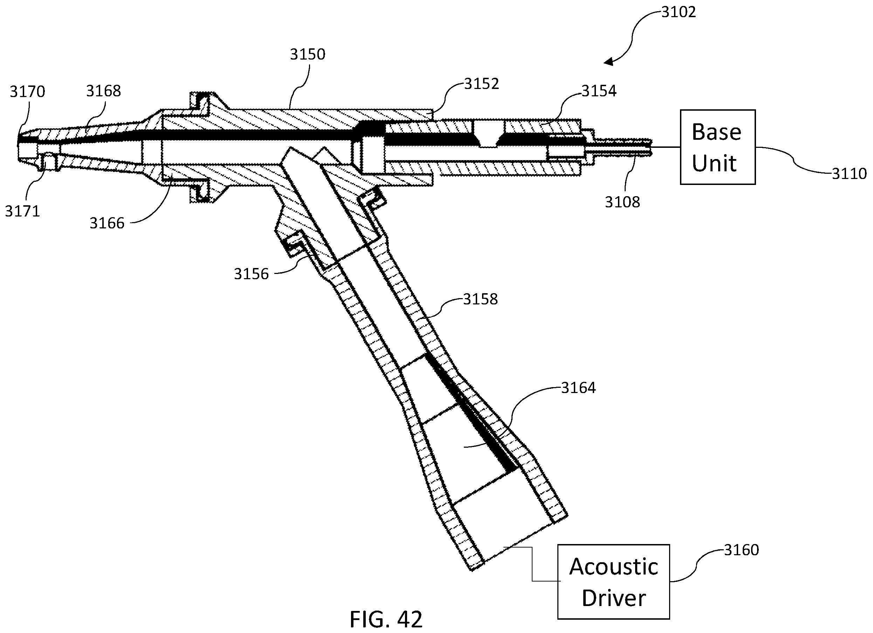

FIG. 42 is a diagrammatic view of the illustrative hand unit 3102 of FIG. 38. The hand unit 3102 illustratively includes a body or housing 3150 including an air inlet 3152 (on the right hand side in FIG. 42). Air pulses from the base unit 3110 are transferred to the inlet 3152 of the hand unit 3102 via the flexible tube 3108. An adjustable pressure limiting (APL) valve 3154 can be mounted to, and fluidly coupled to, the inlet 3152. The body 3150 further includes a downwardly extending, angled extension 3156. An acoustic driver (e.g., an acoustic transducer or speaker) 3160 is connected to a side arm 3158 including an internal chamber 3164 and coupled to the extension 3156. Illustratively, the side arm 3158 extends 60 degrees from the inlet 3152, and the chamber 3164 (illustratively having an internal diameter of 15 mm) receives at least a portion of the acoustic driver 3160.

The body 3150 of the hand unit 3102 illustratively includes an outlet 3166 supporting a converging nozzle 3168 that connects to a mouthpiece 3170. A pressure transducer 3171 can be mounted at the edge of the nozzle 3168.

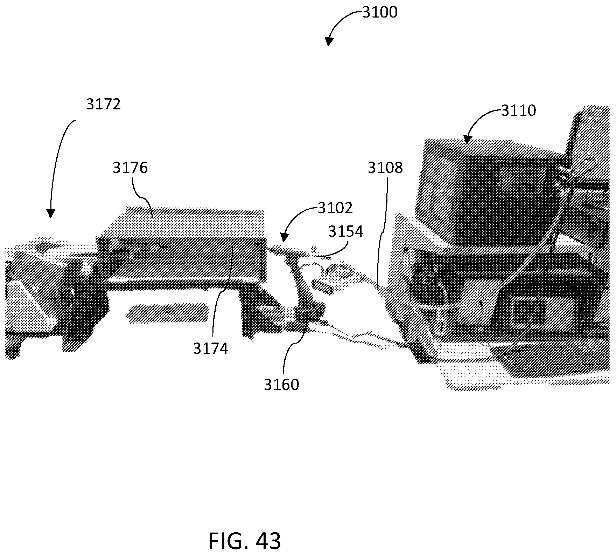

FIG. 43 is a is a schematic view of a test setup for testing the effects of oscillating air pressure pulses and acoustic oscillations on a simulated airway, the test setup including the airway clearance device 3100 of FIG. 38. The right the base unit 3110 is shown with the flexible tube 3108 connecting it to the hand unit 3102. The length of the tube 3108 does not appear to affect the results of airway clearing (clinical effect-CE). The APL 3154 and the acoustic driver 3160 are shown mounted on the hand unit 3102. The mouthpiece 3170 of the hand unit 3102 is fluidly coupled to a passive lung simulator 3172 via a tube 3174, simulating a patient airway. More particularly, the tube 3174 is terminated by the passive lung simulator 3172. The tube 3174 is received in a box or case 3176 in order to obtain clear images of the airway clearance process without external light interference.

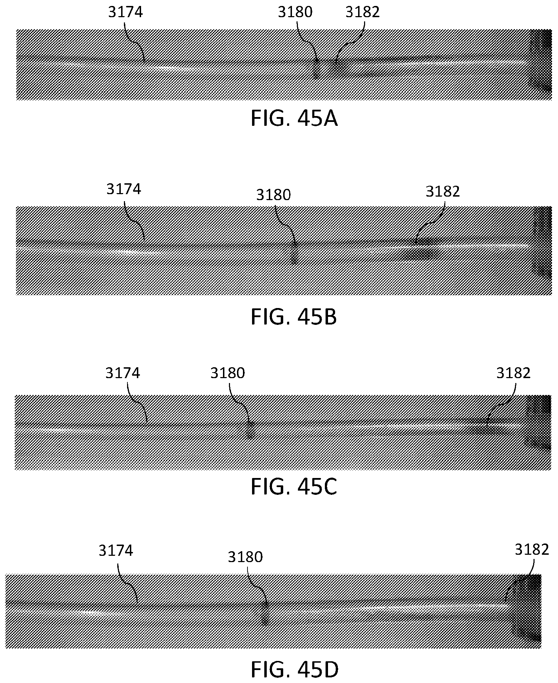

As further detailed herein, a mucus simulant 3182 (FIG. 45A) is utilized in the tube 3174 of the test setup of FIG. 43. In the illustrative embodiment, the mucus simulant 3182 is polyethylene oxide with concentrations of between 1.5 percent and 3 percent.

Clearance tests were conducted with the test setup of FIG. 43 having a test tube 3174 of 11 inches (28 cm) in length and ¼ inch (0.635 cm) in diameter. The mucus simulant 3182 was placed at different distances from the mouthpiece 3170. The parameters tested included: flow pressure of 20-50 cmH2O, flow pulsations at 5 Hz, acoustic amplitude of 05-0.8V and frequency range of 100-700 Hz. For each condition, the pressure at the mouthpiece 3170 and at the end of the test tube 3174 before the passive lung 3172 were recorded. Videos of the mucus simulant 3182 movement were taken, synchronized with the pressure traces.

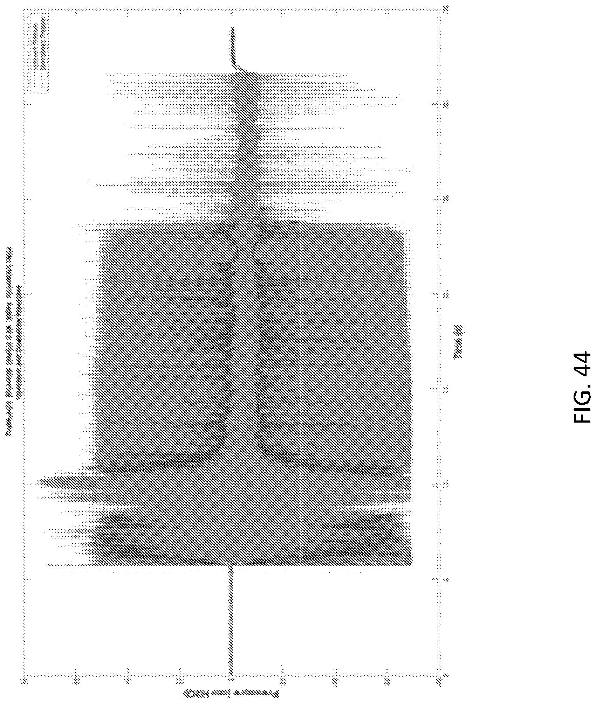

An example of the pressure traces taken at flow pressure of 20 cm H2O, 5 Hz flow pulsations, acoustic amplitude of 0.5V and 300 Hz frequency is shown in FIG. 44. The corresponding snapshots from the clearance video taken at 1, 5, 10 and 15 seconds is shown in FIGS. 45A-45D, respectively. The mucus simulant 3182 was cleared within approximately 10 seconds. The center trace in Fig. 44 shows the pressure at the simulated lung 3172 and the outer trace shows the pressure at the mouthpiece 3170 location. It shows that the pressure at the simulated lung 3172 was higher than at the mouthpiece 3170, thus pushing the mucus simulant 3182 into the hand unit 3102.

With further reference to FIGS. 45A-45D, the mucus simulant 3182 is shown relative to the position of a reference line 3180. FIG. 45A illustrates the mucus simulant 3182 proximate the reference line 3180 at a time of 1 second. FIG. 45B shows that after 5 seconds the mucus simulant 3182 moved about half-way towards the hand unit 3102. FIG. 45C shows that the mucus simulant 3182 reached the hand unit 3102 at 10 seconds. At 15 seconds the mucus simulant 3182 was completely inside the hand unit 3102 (as shown in FIG. 45D).

When placed at 25 cm from the mouthpiece 3170, the mucus simulant 3182 cleared within less than 2 seconds, at 500 Hz frequency for acoustic amplitude of 0.5-0.8V for flow pressure of 20 cm H2O and and 5 Hz flow pulsations. When placed at 15 cm from the mouthpiece 3170, the simulant 3182 cleared within less than 10 sec, at 300 Hz frequency for acoustic amplitude of 0.5-0.8V for flow pressure of 20 cm H2O and 5 Hz flow pulsations. When placed at 10 cm from the mouthpiece 3170, the simulant 3182 cleared within less than 2 sec, at 300 Hz frequency for acoustic amplitude 0.4 and 0.6-0.8V and 1 minute at 0.5V, for flow pressure of 20 cm H2O and 5 Hz flow pulsations. When placed at 5 cm from the mouthpiece 3170, the simulant 3182 cleared within less than 3 sec, at 300 and 400 Hz frequency for acoustic amplitude 0.4-0.5V for flow pressure of 20 cm H2O and and 5 Hz flow pulsations.

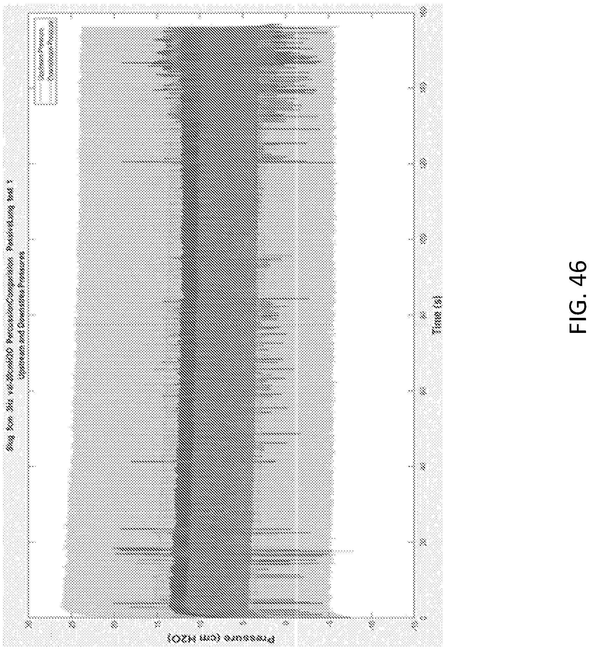

FIG. 46 is a graph showing pressure traces from tests with conventional devices. Experiments using the Vibralung device, of the type detailed above, produced no oscillation or breakdown of the mucus simulant 3182 for all three settings of the same. Tests with and without the mouthpiece 3170 yielded the same results. Experiments using the Percussionaire® IPV device, of the type detailed above, were conducted for mucus simulant 3182 positions 5 cm and 10 cm, with pressures of 5 cm H2O and 10 cm H2O, respectively.

According to the data obtained, plain percussive air pulsations are shown to produce undesirable results, such that no movements or sudden downstream and further delayed pause in oscillatory behavior. Moreover, some cases produced incomplete simulant 3182 breakdowns wherein partial clearance for passing air was developed. (Note that three settings of frequency were considered as different power levels (i.e., low = 1.67 Hz, medium = 3.33 Hz, and high = 5 Hz). Fig. 46 provides pressure plots which show a visual indication of the higher pressure at the hand unit 3102 relative to downstream pressure which led to the undesirable results.

The present invention does not extend beyond the scope of the attached claims.

## Claims

1. A device (100) for treating airways, the device comprising:

   a base unit (110) comprising a housing (180), the housing containing an air pump (182), a flow pulsating element (156) configured to alter the continuous airflow stream from the air pump according to a predetermined pattern, said flow pulsating element being a three-way solenoid valve, which allows a downstream flow of air during a first part of the device cycle and an upstream flow of air during a second part of the device cycle, which allows the device (100) to send pulsations into a patient during an inhalation event while also allowing for expiration of air during an exhalation event, or two high-speed one-directional valves consisting of a first directional valve for inflow and a second directional valve for outflow, a circuit board assembly, and a memory;
   a hand unit (102, 1600, 1700) operatively coupled to the base unit, the hand unit comprising:

   a housing (104, 1602, 1702) containing an acoustic transducer (1616, 1716) configured to apply acoustic oscillations according to a predetermined pattern, a central duct (1604, 1704) in fluid communication with the base unit, and an acoustic duct (1614, 1714) in fluid communication with the central duct; and
   a mouthpiece (106, 1608, 1708) coupled to the housing.

2. The device of claim 1, wherein the base unit (110) is configured to be mobile and worn by a patient.

3. The device of claim 2, further comprising a chest rack (112) configured to hold the hand unit (102, 1600, 1700) in a stationary position to facilitate hands-free use of the device.

4. The device of claim 1, further comprising a nebulizer coupled to the mouthpiece (106, 1608, 1708) of the device.

5. The device of claim 1, wherein the device is configured for wireless communication.

6. The device of claim 1, wherein the base unit (110) further comprises a display screen (114), preferably a touch screen, more preferably a liquid crystal display touch screen.

7. The device of claim 1, wherein the flow pulsating element (156) is configured to alter the continuous airflow stream from the air pump (182) according to a square pattern or a sinusoidal pattern.

8. The device of claim 1, wherein the flow pulsating element (156) creates a centrifuging vortex flow, wherein the axis of rotation is in the direction of the airflow.

9. The device of claim 1, wherein the flow pulsating element (156) creates air pressure pulses or vibrations in a frequency range between about 5 Hz and about 700 Hz.

10. The device of claim 1, further comprising a positive expiratory pressure (PEP) mechanism for creating back-flow resistance.

11. A system for using the device (100) of claim 1, the system comprising:

    the device (100) of claim 1;
    a first personal electronic application in communication with the device (100) and in communication with a cloud-based server (136); and
    a second personal electronic application in communication with the cloud-based server.

12. The system of claim 11, wherein the second personal electronic application is configured to control a variety of parameters of the device from a remote location via the cloud-based server (136) and the first personal electronic application.

## Patentansprüche

1. Vorrichtung (100) zum Behandeln von Atemwegen, wobei die Vorrichtung umfasst:

   eine Basiseinheit (110), umfassend ein Gehäuse (180), wobei das Gehäuse eine Luftpumpe (182), ein Strömungspulsierungselement (156), das konfiguriert ist, um den kontinuierlichen Luftstrom von der Luftpumpe gemäß einem vorbestimmten Muster zu verändern, wobei das Strömungspulsierungselement ein Drei-Wege-Magnetventil ist, das während eines ersten Abschnitts des Vorrichtungszyklus einen stromabwärts gerichteten Luftstrom und während eines zweiten Abschnitts des Vorrichtungszyklus einen stromaufwärts gerichteten Luftstrom zulässt, wodurch die Vorrichtung (100) in der Lage ist, Pulsationen während eines Inhalationsereignisses in einen Patienten zu senden, während sie zugleich das Ausatmen der Luft während eines Exhalationsereignisses ermöglicht, oder zwei Hochgeschwindigkeits-Einwegventile, bestehend aus einem ersten Richtungsventil für den Einstrom und einem zweiten Richtungsventil für den Ausstrom, eine Leiterplattenanordnung, und einen Speicher einschließt;
eine Hand-einheit (102, 1600, 1700), die betriebsmäßig mit der Basiseinheit gekoppelt ist, wobei die Handeinheit umfasst:

ein Gehäuse (104, 1602, 1702), das einen akustischen Wandler (1616, 1716), der konfiguriert ist, um akustische Oszillationen gemäß einem vorbestimmten Muster anzuwenden, einen zentralen Kanal (1604, 1704), der fluidisch mit der Basiseinheit verbunden ist, und einen akustischen Kanal (1614, 1714), der fluidisch mit dem zentralen Kanal verbunden ist, einschließt; und
ein Mundstück (106, 1608, 1708), das mit dem Gehäuse gekoppelt ist.

2. Vorrichtung nach Anspruch 1, wobei die Basiseinheit (110) konfiguriert ist, um mobil zu sein und von einem Patienten getragen zu werden.

3. Vorrichtung nach Anspruch 2, ferner umfassend einen Brusthalter (112), der konfiguriert ist, um die Handeinheit (102, 1600, 1700) in einer stationären Position zu halten, um eine freihändige Benutzung der Vorrichtung zu ermöglichen.

4. Vorrichtung nach Anspruch 1, ferner umfassend einen Vernebler, der mit dem Mundstück (106, 1608, 1708) der Vorrichtung gekoppelt ist.

5. Vorrichtung nach Anspruch 1, wobei die Vorrichtung für drahtlose Kommunikation konfiguriert ist.

6. Vorrichtung nach Anspruch 1, wobei die Basiseinheit (110) ferner einen Bildschirm (114) umfasst, vorzugsweise einen Touchscreen, mehr bevorzugt einen Flüssigkristall-Touchscreen.

7. Vorrichtung nach Anspruch 1, wobei das Strömungspulsierungselement (156) konfiguriert ist, um den kontinuierlichen Luftstrom von der Luftpumpe (182) gemäß einem quadratischen Muster oder einem sinusförmigen Muster zu verändern.

8. Vorrichtung nach Anspruch 1, wobei das Strömungspulsierungselement (156) einen zentrifugierenden Wirbelstrom erzeugt, wobei die Drehachse in Richtung des Luftstroms verläuft.

9. Vorrichtung nach Anspruch 1, wobei das Strömungspulsierungselement (156) Luftdruckimpulse oder Vibrationen in einem Frequenzbereich zwischen etwa 5 Hz und etwa 700 Hz erzeugt.

10. Vorrichtung nach Anspruch 1, ferner umfassend einen positiven exspiratorischen Druckmechanismus (PEP) zum Erzeugen eines Rückflusswiderstands.

11. System zur Verwendung der Vorrichtung (100) nach Anspruch 1, wobei das System umfasst:

die Vorrichtung (100) nach Anspruch 1;
eine erste persönliche elektronische Anwendung, die mit der Vorrichtung (100) und mit einem cloudbasierten Server (136) in Kommunikation steht; und
eine zweite persönliche elektronische Anwendung, die mit dem cloudbasierten Server in Kommunikation steht.

12. System nach Anspruch 11, wobei die zweite persönliche elektronische Anwendung konfiguriert ist, um eine Vielzahl von Parametern der Vorrichtung von einem entfernten Standort über den cloudbasierten Server (136) und die erste persönliche elektronische Anwendung zu steuern.

## Revendications

1. Dispositif (100) pour le traitement des voies respiratoires, le dispositif comprenant :

une unité de base (110) comprenant un boîtier (180), le boîtier contenant une pompe à air (182), un élément de pulsation d'écoulement (156) conçu pour modifier le flux d'écoulement d'air continu provenant de la pompe à air conformément à un motif prédéterminé, ledit élément de pulsation d'écoulement étant une électrovanne à trois voies, qui permet un écoulement d'air en aval pendant une première partie du cycle du dispositif et un écoulement d'air en amont pendant une seconde partie du cycle du dispositif, qui permet au dispositif (100) d'envoyer des pulsations dans un patient au cours d'une inhalation tout en permettant l'expiration de l'air au cours d'une expiration, ou deux vannes unidirectionnelles à grande vitesse constituées d'une première valve directionnelle pour l'entrée de l'écoulement et d'une seconde valve directionnelle pour la sortie de l'écoulement, d'un ensemble carte de circuit imprimé et d'une mémoire ;

une unité manuelle (102, 1600, 1700) accouplée de manière fonctionnelle à l'unité de base, l'unité manuelle comprenant :

un boîtier (104, 1602, 1702) contenant un transducteur acoustique (1616, 1716) configuré pour appliquer des oscillations acoustiques conformément à un motif prédéterminé, un conduit central (1604, 1704) en communication fluidique avec l'unité de base, et un conduit acoustique (1614, 1714) en communication fluidique avec le conduit central ; et

un embout buccal (106, 1608, 1708) accouplé au boîtier.

2. Dispositif selon la revendication 1, dans lequel l'unité de base (110) est conçue pour être mobile et portée par un patient.

3. Dispositif selon la revendication 2, comprenant en outre un support pour poitrine (112) conçu pour maintenir l'unité manuelle (102, 1600, 1700) dans une position fixe afin de faciliter l'utilisation mains libres du dispositif.

4. Dispositif selon la revendication 1, comprenant en outre un nébuliseur accouplé à l'embout buccal (106, 1608, 1708) du dispositif.

5. Dispositif selon la revendication 1, dans lequel le dispositif est configuré pour la communication sans fil.

6. Dispositif selon la revendication 1, dans lequel l'unité de base (110) comprend en outre un écran d'affichage (114), de préférence un écran tactile, plus préférablement un écran tactile à cristaux liquides.

7. Dispositif selon la revendication 1, dans lequel l'élément de pulsation d'écoulement (156) est conçu pour modifier le flux d'écoulement d'air continu provenant de la pompe à air (182) conformément à un motif carré ou à un motif sinusoïdal.

8. Dispositif selon la revendication 1, dans lequel l'élément de pulsation d'écoulement (156) crée un écoulement tourbillonnaire centrifuge, dans lequel l'axe de rotation est dans la direction de l'écoulement d'air.

9. Dispositif selon la revendication 1, dans lequel l'élément de pulsation d'écoulement (156) crée des impulsions de pression d'air ou des vibrations dans une gamme de fréquences comprise entre environ 5 Hz et environ 700 Hz.

10. Dispositif selon la revendication 1, comprenant en outre un mécanisme de pression expiratoire positive (PEP) pour la création d'une résistance au reflux.

11. Système permettant l'utilisation du dispositif (100) selon la revendication 1, le système comprenant : le dispositif (100) selon la revendication 1 ;

une première application électronique personnelle en communication avec le dispositif (100) et en communication avec un serveur en nuage (136) ; et

une seconde application électronique personnelle en communication avec le serveur en nuage.

12. Système selon la revendication 11, dans lequel la seconde application électronique personnelle est configurée pour commander une variété de paramètres du dispositif à partir d'un emplacement distant par l'intermédiaire du serveur en nuage (136) et de la première application électronique personnelle.

FIG. 1A

FIG. 1B

FIG. 1C

FIG. 2A

FIG. 2B

EP 4 426 402 B1

FIG. 3A

FIG. 3B

25

138   100

BT/Wifi Communication +
Bi-directional data transfer

134

Patient

Smartphone

Treatment records & Patient adherence

BT/Wifi Communication +
Bi-directional data transfer

136

Caregiver

Cloud Based system

140

Prescription &
effective treatment

FIG. 4

**Disease Management** —142
Track & Record different protocol treatment, medication, lungs condition sensing

**Adherence Tracking** —144
Treatment stages and progress, get feedback on device usage and your lungs condition

**Remote Monitoring** —146
Track patient progress with a direct & sending automatic notification

**Personalised Treatment** —148
Modify the treatment protocol, medications according to disease progress and patient condition

**Device-to-Cloud** —150
A real-time data storage for accessible usage & monitoring

134

Reports  Notifications  Settings

Protocol
Session time
Usage

Tracking  Protocol

○ Smartphone

FIG. 5

FIG. 6A

| Requirement | Value | Unit |
|---|---|---|
| Inlet air pressure @ 50-100 LPM | 15-50 | cm $H_2O$ |
| Outlet air pressure amplitude | 5-50 | cm $H_2O$ |
| Air pulse frequencies | 5-700 | Hz |
| Outlet acoustical amplitude | 30-105 | dB |
| Outlet acoustical pulse frequency | 200-10,000 | Hz |
| Outlet pressure during PEP maneuvers | 2-25 | cm $H_2O$ |

FIG. 6B

| | Generation | | Diameter, cm | Length, cm | Number | Total cross-sectional area, $cm^2$ |
|---|---|---|---|---|---|---|
| Conducting zone | trachea | 0 | 1.80 | 12.0 | 1 | 2.54 |
| | bronchi | 1 | 1.22 | 4.8 | 2 | 2.33 |
| | | 2 | 0.83 | 1.9 | 4 | 2.13 |
| | | 3 | 0.56 | 0.8 | 8 | 2.00 |
| | bronchioles | 4 | 0.45 | 1.3 | 16 | 2.48 |
| | | 5 | 0.35 | 1.07 | 32 | 3.11 |
| | terminal bronchioles | 16 | 0.06 | 0.17 | $6 \times 10^4$ | 180.0 |
| transitional and respiratory zones | respiratory bronchioles | 17 18 19 | 0.05 | 0.10 | $5 \times 10^5$ | $10^3$ |
| | alveolar ducts T3 T2 T1 | 20 21 22 | | | | |
| | alveolar sacs T | 23 | 0.04 | 0.05 | $8 \times 10^6$ | $10^4$ |

FIG. 7A

FIG. 7B

EP 4 426 402 B1

FIG. 8

1056                                                    1054

FIG. 9A

Mouth side        1056                         1054                        Lungs side

FIG. 9B

1054

FIG. 9C

1054

1050                                            FIG. 9D                                      1052

FIG. 10A

FIG. 10B

FIG. 10C

FIG. 11A

FIG. 11B

FIG. 11C

FIG. 11D

FIG. 12A

FIG. 12B

FIG. 12C

FIG. 12D

FIG. 13

FIG. 14

Shop Air Supply — 2004

2010  2006  2008

2016  2018  Pressure Sensor -1

2014  2017

Microphone (Perpendicular, 2.54 cm) — 2032

1022  2026  Test Section

31cm

2029  Camera  2024

2020

2012  Controller

2028  Pressure Sensor -2

2030

2000

122cm

*a. With Increasing Speaker Amplitude @ 6Hz*

| Supply Pressure (Cm of H20) | Speaker Amp (V) | 3/2 freq (Hz) | Sound (dB) |
|---|---|---|---|
| 30 | 0.4 | 6 | 86.0099 |
| 30 | 0.5 | 6 | 86.4529 |
| 30 | 0.6 | 6 | 87.0007 |
| 30 | 0.7 | 6 | 86.9886 |
| 30 | 0.8 | 6 | 88.1262 |

FIG. 15A

*b. With Increasing Valve Frequency @ 0.4 V*

| Supply Pressure (Cm of H20) | Speaker Amp (V) | 3/2 freq (Hz) | Sound (dB) |
|---|---|---|---|
| 30 | 0.4 | 12 | 84.0541 |
| 30 | 0.4 | 15 | 83.4104 |
| 30 | 0.4 | 20 | 82.8664 |
| 30 | 0.4 | 30 | 82.8457 |

FIG. 15B

| Pressure (cm of H20) | Sol Freq (Hz) | Speaker Amp (V) | Sound (dB) |
|---|---|---|---|
| 20 | 1.667 | 0.2 | 73.951 |
| 20 | 6 | 0.2 | 73.7863 |
| 20 | 12 | 0.2 | 73.3108 |
| 20 | 1.667 | 0.4 | 82.0463 |
| 20 | 6 | 0.4 | 79.1654 |
| 20 | 12 | 0.4 | 78.152 |
| 20 | 1.667 | 0.8 | 85.9076 |
| 20 | 6 | 0.8 | 84.5329 |
| 20 | 12 | 0.8 | 83.681 |

FIG. 15C

| Pressure (cm of H20) | Sol Freq (Hz) | Speaker Amp (V) | Sound (dB) |
|---|---|---|---|
| 40 | 1.667 | 0.2 | 74.9351 |
| 40 | 6 | 0.2 | 74.4631 |
| 40 | 12 | 0.2 | 72.6783 |
| 40 | 1.667 | 0.4 | 79.5493 |
| 40 | 6 | 0.4 | 79.638 |
| 40 | 12 | 0.4 | 78.5786 |
| 40 | 1.667 | 0.8 | 85.3316 |
| 40 | 6 | 0.8 | 84.0879 |
| 40 | 12 | 0.8 | 83.2556 |

FIG. 15D

FIG. 16

2132

2117

2108

2116

2100

FIG. 17B

FIG. 17A

FIG. 18B

FIG. 18A

Speaker-nofoam-1000Hz-0.4-0.8

2200

FIG. 19A

Speaker-nofoam-1000Hz-0.4-0.8

2202

FIG. 19B

EP 4 426 402 B1

FIG. 19D

FIG. 19C

EP 4 426 402 B1

**Speaker-nofoam-1000Hz-0.4-0.8**

Sound Pressure, K Pa

Time, s

2400

FIG. 19E

**Speaker-nofoam-1000Hz-0.4-0.8**

Sound Level, dB

Amplitude, a

2402

FIG. 19F

Speaker-WithFoam-1000Hz-0.4-0.8

2502

**FIG. 19H**

Speaker-WithFoam-1000Hz-0.4-0.8

2500

**FIG. 19G**

46

FIG. 21B

FIG. 21A

FIG. 20B

FIG. 20A

FIG. 23B

FIG. 23A

FIG. 22B

FIG. 22A

speaker amplitude 0.4, 3/2 V frequency 12Hz

2602a

FIG. 24A

speaker amplitude 0.4, 3/2 V frequency 12Hz

2602b

FIG. 24B

speaker amplitude 0.4, 3/2 V frequency 12Hz

2604a

FIG. 25A

speaker amplitude 0.4, 3/2 V frequency 12Hz

2604b

FIG. 25B

EP 4 426 402 B1

2702a   2702b

speaker amplitude 0.4, 3/2 V frequency 15Hz

FIG. 26A

speaker amplitude 0.4, 3/2 V frequency 15Hz

FIG. 26B

speaker amplitude 0.4, 3/2 V frequency 15Hz

2704a

FIG. 27A

speaker amplitude 0.4, 3/2 V frequency 15Hz

2704b

FIG. 27B

2802a

2802b

speaker amplitude 0.4, 3/2 V frequency 30Hz

FIG. 28A

speaker amplitude 0.4, 3/2 V frequency 20Hz

FIG. 28B

speaker amplitude 0.4, 3/2 V frequency 20Hz

2804a

FIG. 29A

speaker amplitude 0.4, 3/2 V frequency 20Hz

2804b

FIG. 29B

speaker amplitude 0.4, 3/2 V frequency 30Hz

FIG. 30A

speaker amplitude 0.4, 3/2 V frequency 30Hz

FIG. 30B

speaker amplitude 0.4, 3/2 V frequency 30Hz

FIG. 31A

speaker amplitude 0.4, 3/2 V frequency 30Hz

FIG. 31B

FIG. 32

FIG. 33

EP 4 426 402 B1

IPV Percussionaire

IPV Perc 40cmH2O High

Upstream Pressure

Downstream Pressure

FIG. 34

FIG. 35A

FIG. 35B

Aeroself Device - combined air pulses and acoustics
SP-30cmH2O ValFreq-12 SpFreq 900 SpAmp-0.4 No-Foam

FIG. 36

FIG. 37

FIG. 38

FIG. 39

FIG. 40

EP 4 426 402 B1

FIG. 41

FIG. 42

FIG. 43

FIG. 44

FIG. 45A

FIG. 45B

FIG. 45C

FIG. 45D

FIG. 46

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019140333 A1
- US 20130186399, Ephraim Gutmark,